# EUROPÄISCHE PATENTSCHRIFT

(11) **EP 3 793 496 B1**
(45) Veröffentlichungstag und Bekanntmachung des Hinweises auf die Patenterteilung: **20.07.2022**
(21) Anmeldenummer: 19727594.4
(22) Anmeldetag: 17.05.2019
(51) Int. Cl.: A61F 13/49, A61F 13/491, A61F 13/494, A61F 13/496

(54) **INKONTINENZARTIKEL**
INCONTINENCE PRODUCT
ARTICLE POUR L'INCONTINENCE

(30) Priorität: 18.05.2018 DE 102018112120
(43) Veröffentlichungstag der Anmeldung: 24.03.2021
(73) Patentinhaber: Paul Hartmann AG, 89522 Heidenheim (DE)
(72) Erfinder: BUCH, Tamara, 89077 Ulm (DE); EILERS, Joerg, 89179 Beimerstetten (DE); KESSELMEIER, Ruediger, 89233 Neu-Ulm (DE); BEYRLE, Andreas, 89564 Nattheim (DE); ROHRBACHER, Agnes, 89520 Heidenheim (DE); SCHMIDT, Ann-Cathrin, 06667 Weissenfels (DE)
(74) Vertreter: DREISS Patentanwälte PartG mbB
(86) Internationale Anmeldenummer: PCT/EP2019/062875
(87) Internationale Veröffentlichungsnummer: WO 2019/219945

(56) Entgegenhaltungen:
- WO-A1-00/47152
- WO-A1-2016/149592
- DE-A1-102005 030 182
- DE-A1-102007 002 290

## Beschreibung

Die Erfindung betrifft einen Inkontinenzartikel in Höschenform, also einen "pull-up"-Artikel, für die Aufnahme von Körperausscheidungen mit einem vorderen Bauchabschnitt und einem hinteren Rückenabschnitt, die in einer Längsrichtung voneinander beabstandet sind und die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der sich in der Längsrichtung zwischen Bauchabschnitt und Rückenabschnitt erstreckt und an den Bauchabschnitt und an den Rückenabschnitt in einem jeweiligen Überlappungsbereich unlösbar angefügt ist, wobei der Schrittabschnitt, der Bauchabschnitt und der Rückenabschnitt gemeinsam jeweilige Beinöffnungen des Inkontinenzartikels begrenzen, wobei der Bauchabschnitt und der Rückenabschnitt in Quer- oder Hüftumfangsrichtung elastisch dehnbar ausgebildet sind, wobei der Schrittabschnitt beidseits eine seitliche Auslaufsperre bildende und beidseits entlang der Längserstreckung des Absorptionskörpers verlaufende Cuffelemente aufweist, die zumindest entlang einer Cuffsockellinie an der körperzugewandten Seite des Artikels festgelegt sind und einen unbefestigten freien Längsrand aufweisen, mit dem sie sich von der körperzugewandten Seite des Artikels emporerheben können und so die jeweilige seitliche Auslaufsperre bilden.

Ein derartiger dreikomponentiger Inkontinenzartikel, bei dem der Bauchabschnitt, der Rückenabschnitt und der Schrittabschnitt diese drei Komponenten bilden, ist beispielsweise bekannt aus DE 10 2007 055 628 A1.

Dieser Inkontinenzartikel in Höschenform soll als ein diskretes, im Sinne von unauffällig vom Anwender zu tragendes Produkt weiterentwickelt werden, welches gleichwohl wesentlichen an einen Inkontinenzartikel gestellten Anforderungen, wie u.a. die des Auslaufschutzes gerecht wird. Vor allem für Anwender, die unter einer leichteren Form der Inkontinenz leiden und durchaus mobil sind, ist die Bereitstellung von funktionellen aber dennoch komfortablen und ein gutes Tragegefühl unterstützenden Inkontinenzartikeln wichtig. Eine Ausgewogenheit an Funktionalität und Komfort ist vor allem weiblichen Anwendern von Inkontinenzartikeln ein vorrangiges Anliegen. Mit Blick auf die Diskretion soll dieser Inkontinenzartikel in Höschenform insbesondere auch als schmales oder schlankes Produkt mit eher kleinen Abmessungen weiterentwickelt werden, obschon es für Erwachsene konzipiert ist, es sich also nicht um eine Trainingpant für Kinder handelt. Der Inkontinenzartikel soll vorzugsweise für die Verwendung durch Frauen ausgebildet werden.

Diese Aufgabe wird erfindungsgemäß durch einen Inkontinenzartikel mit den Merkmalen des Anspruchs 1 gelöst.

Es wurde also erfindungsgemäß von herkömmlichen bei derartigen Inkontinenzartikeln typischerweise eingesetzten elastifizierten Cuffsystemen Abstand genommen. Diese vorbekannten Cuffsysteme waren typischerweise so ausgebildet, dass ein jeweiliges Cuffelement in der Regel im Bereich seines freien emporerhebbaren Längsrands elastifiziert ist und der emporerhebbare Bereich des Cuffelements, also die aufstehbare Cuffhöhe, zudem oftmals sehr groß bemessen ist. Im Ergebnis verdecken deshalb die herstellerseitig auf die Oberseite des Absorptionskörpers eingeschlagenen Cuffelemente große Oberflächenbereiche des Absorptionskörpers, was bei breit bauenden Schrittabschnitten und Absorptionskörpern auch eher unproblematisch ist. Bei eher schmal bauenden Produkten kann dies aber zu Komplikationen führen. Es wurde erkannt, dass durch Elastifizierung in Cuffelementen induzierte Zugkräfte in nachteiliger Weise zu Überfaltungen und Verknautschen des Absorptionskörpers führen können. Zudem bringt die Elastifizierung von Cuffelementen auch Fältelungen und lokale Versteifungen in die Cuffelement-Materialien ein, die vom Anwender als unangenehm empfunden werden und auch Hautreizungen verursachen können, zumal wenn diese Fältelungen und Versteifungen vermehrt in den genital sensiblen Bereichen vorhanden sind.

Mit der vorliegenden Erfindung wird deshalb vorgeschlagen, von einer derartigen Elastifizierung Abstand zu nehmen, was sich insbesondere bei schmal bauenden Artikeln und/oder insbesondere bei Artikeln für Frauen als vorteilhaft erweist, weil solchenfalls keine durch eine Elastifizierung hervorgerufenen Störeffekte auftreten. Insbesondere vermitteln die Cuffelemente ein angenehmes Tragegefühl. Zudem können sich diese Cuffelemente aufgrund der durch die Umfaltung des Vliesmaterialabschnitts bedingten Doppellagigkeit besser ihrer Umgebung anpassen, so beispielsweise bei Druck auf die Faltkante sich in gewisser Weise in ihrem Querschnitt ausbreiten.

Es wurde weiter erkannt, dass vor allem ein solcher dreikomponentiger Inkontinenzartikel in Höschenform - gebildet aus den drei separaten Komponenten Schrittabschnitt, Bauchabschnitt und Rückenabschnitt - geeignet ist, ein diskretes Produkt mit Cuffelementen frei von Elastifizierungsmittel bereitstellen zu können: Das Emporerheben der Cuffelemente wird zum einen durch die Überlappung des Schrittabschnitts mit einem in Quer- oder Hüftumfangsrichtung elastisch dehnbar ausgebildeten Bauchabschnitt und Rückenabschnitt vorteilhaft unterstützt, indem im Anwendungszustand des Inkontinenzartikels durch die Zugwirkung aus Bauch- und Rückenabschnitt auf die Überlappungsbereiche mit dem Schrittabschnitt das Ausbilden einer "bootförmigen" Konfiguration des Schrittabschnitts gefördert und damit auch die Cuffelemente emporerhoben werden, zumindest im Bereich der Quermittelachse des Artikels.

Zum anderen tragen die im Schrittabschnitt vorhandenen und den Cuffelementen auswärts direkt zugeordneten Schrittelastifizierungsmittel ebenso vorteilhaft zur Ausformung einer bootförmigen Konfiguration bei. Der Schrittabschnitt als separate Komponente kann somit entsprechend den Anforderungen bedarfsgerecht ausgestaltet werden.

Der umgefaltete und das Cuffelement bildende Vliesmaterialabschnitt weist außerhalb des umgefalteten Bereichs vorzugsweise zumindest einen Vliesmaterialteilabschnitt auf, welcher sich insbesondere in Querrichtung auswärts, und dabei insbesondere bis zu einem Längsrand des Schrittabschnitts erstreckt.

Im Falle von zwei Vliesmaterialteilabschnitten können diese hinsichtlich ihrer Quererstreckung unterschiedlich sein. Alternativ ist auch eine weitgehend gleiche Quererstreckung denkbar, wenngleich eine unterschiedliche Quererstreckung bevorzugt ist, da eine Reduktion an Materiallagen auch zu einer Diskretion des Inkontinenzartikels beiträgt. Im Falle der unterschiedlichen Quererstreckung der Vliesmaterialteilabschnitte ist der längere Vliesmaterialteilabschnitt vorzugsweise der körperzugewandten Seite angeordnet.

Der Vliesmaterialabschnitt zur Bereitstellung der Cuffelemente ist vorzugsweise aus einem hydrophoben Vliesmaterial gebildet.

Das Flächengewicht für den Vliesmaterialabschnitt beträgt vorzugsweise 6-20 g/m², insbesondere 6-15 g/m², weiter insbesondere 8-15 g/m², weiter insbesondere 10-15 g/m².

In Weiterbildung der Maßnahme, zumindest die emporerhebbaren Bereiche der Cuffelemente frei von elastifizierenden Mitteln auszubilden, wird weiter vorgeschlagen, die Cuffhöhe des emporerhebbaren Bereichs eher gering auszubilden, dabei abgestimmt auf die weiteren funktionellen Bereiche innerhalb des Schrittabschnitts, damit eine vorteilhafte Kombination an Funktionalität und Komfort erhalten wird.

Vorzugsweise ist eine Quererstreckung (H) des von der körperzugewandten Seite des Artikels emporerhebbaren Bereichs des jeweiligen Cuffelements, also die Cuffhöhe, höchstens so lang wie eine Quererstreckung (F) des Schrittabschnitts gemessen von der Cuffsockellinie nach außen beträgt. Bevorzugt ist die Cuffhöhe kleiner als die Quererstreckung F des Schrittabschnitts nach außen. Die Cuffhöhe wird außerhalb der Überlappung des Schrittabschnitts mit dem Bauchabschnitt bzw. mit dem Rückenabschnitt bestimmt, also in dem Bereich, wo Bauchabschnitt und Rückenabschnitt voneinander beabstandet sind, der Schrittabschnitt also gewissermaßen als Brückenelement zwischen Bauchabschnitt und Rückenabschnitt wirkt. Damit wird ein unkontrolliertes Überfalten eines äußeren Längsrands des Schrittabschnitts durch die Cuffelemente vermieden.

Insbesondere bevorzugt wird hierfür vorgeschlagen, dass eine Quererstreckung (H) des von der körperzugewandten Seite des Artikels emporerhebbaren Bereichs des jeweiligen Cuffelements, also die Cuffhöhe, wenigstens das 0,3-fache, insbesondere wenigstens das 0,4-fache und höchstens das 1,0-fache, insbesondere höchstens das 0,9-fache, insbesondere höchstens das 0,8-fache und weiter insbesondere höchstens das 0,7-fache einer Quererstreckung (F) des Schrittabschnitts gemessen von der Cuffsockellinie nach außen beträgt, wobei die jeweilige Quererstreckung (H, F) an einer in Querrichtung schmalsten Stelle des Schrittabschnitts bestimmt wird, die in der Längsrichtung zwischen dem Bauchabschnitt und dem Rückenabschnitt liegt. Falls keine schmalste Stelle des Schrittabschnitts vorhanden ist, wie im Falle von geraden parallel verlaufenden Längsrändern des Schrittabschnitts, dann wird als Bemessungsort die Quermittelachse des Inkontinenzartikels herangezogen.

Unter Cuffsockellinie wird die Verbindungsstelle zwischen dem emporerhebbaren Bereich des Cuffelements und einer körperzugewandten Lage des Schrittabschnitts verstanden.

Die Cuffsockellinie kann vorzugsweise aus Ultraschallschweißbereichen und/oder adhäsiven Verbindungsbereichen gebildet werden.

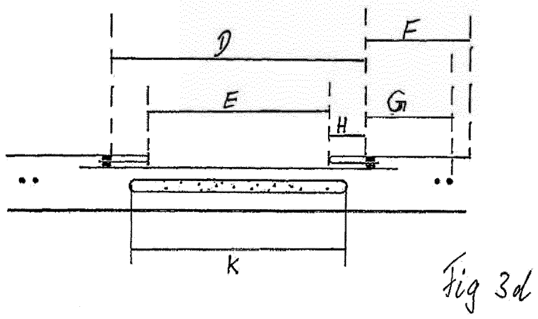

Da die Cuffsockellinie typischerweise eine gewisse Breite im Millimeterbereich aufweisen kann und insbesondere von einzelnen Ultraschallschweißpunkten gebildet ist, die zusammen einen streifenförmigen Pfad bilden, der dann eine Cuffsockellinie einer endlichen Streifenbreite bildet, wird bei der Messung oder Bestimmung der Quererstreckung (F) diese Breite der Cuffsockellinie miteinbezogen, so wie dies in der schematischen Figur 3d dargestellt ist.

An dieser Stelle sei ausgeführt, dass in der vorliegenden Anmeldung jegliche Bemessungsangaben hinsichtlich Quererstreckung, Längserstreckung oder Abstand von Abschnitten oder von ansich beliebigen Komponenten des Artikels im eben ausgebreiteten Zustand der den Artikel bildenden Flachmaterialien bestimmt werden, also gegebenenfalls nach Auftrennung der herstellerseitig schon angebrachten Seitennähte, so dass der betreffende Artikel in die in den Figuren dargestellte ebene Konfiguration gebracht werden kann. Wenn der Artikel beispielsweise durch fadenförmige Elastifizierungsmittel im sogenannten "Stretchbondverfahren" elastifiziert wurde, so werden die Flächenmaterialien, wie in den Figuren angedeutet, derart ausgedehnt betrachtet, wie sie herstellerseitig als Flachmaterialien zugeführt werden oder nachträglich bis zu ihrer natürlichen Ausgangserstreckung ohne Elastifizierungsmittel ausgebreitet und auf eine ebene Fläche aufgelegt werden können. In dieser ebenen Fläche werden dann die Quererstreckungen, Längserstreckungen, Abmessungen oder Abstände ermittelt. Dieser Zustand ergibt sich bei undehnbaren Chassismaterialien auf Vliesbasis oder Vlies/Folien-Verbundbasis auf natürliche Weise wie in den Figuren dargestellt.

Mit einem derart abgestimmten Verhältnis zwischen einer Quererstreckung H des emporerhebbaren Bereichs des Cuffelements und einer Quererstreckung F des davon auswärts verbliebenen Bereichs des Schrittabschnitts wird vorteilhaft das Emporerheben der Cuffelemente unterstützt.

Im vorstehenden Sinne erweist es sich insbesondere weiter als vorteilhaft, dass eine maximale Quererstreckung (H) des von der körperzugewandten Seite des Artikels emporerhebbaren Bereichs des jeweiligen Cuffelements wenigstens 7 mm, insbesondere wenigstens 10 mm, insbesondere wenigstens 12 mm und höchstens 30 mm, insbesondere höchstens 25 mm, insbesondere höchstens 20 mm und weiter insbesondere höchstens 18 mm und insbesondere 15 mm beträgt. Es geht hier also um die maximale Cuffhöhe, wobei die jeweilige Quererstreckung (H) an einer jeweiligen Stelle des Schrittabschnitts bestimmt wird, die in der Längsrichtung zwischen dem Bauchabschnitt und dem Rückenabschnitt liegt.

Die Cuffhöhe kann weiter in vorteilhafter Weise an die Position der Schrittelastifizierungsmittel angepasst sein. Hierfür wird insbesondere vorgeschlagen, dass eine Quererstreckung (H) des von der körperzugewandten Seite des Artikels emporerhebbaren Bereichs des jeweiligen Cuffelements, also die Cuffhöhe, wenigstens das 0,50-fache, insbesondere wenigstens das 0,55-fache, insbesondere wenigstens das 0,60-fache, insbesondere wenigstens das 0,65-fache und höchstens das 1,0-fache, insbesondere höchstens das 0,95-fache, insbesondere höchstens das 0,90-fache eines Querabstands (G) zwischen der Cuffsockellinie und eines in Querrichtung äußersten Schrittelastifizierungsmittels (inklusive dessen Quererstreckung oder Breite ) beträgt, wobei die Quererstreckung (H) und der Querabstand (G) an einer in Querrichtung schmalsten Stelle des Schrittabschnitts bestimmt wird, die in der Längsrichtung zwischen dem Bauchabschnitt und dem Rückenabschnitt liegt. Falls keine schmalste Stelle des Schrittabschnitts vorhanden ist, wie im Falle von geraden parallel verlaufenden Längsrändern, dann wird die Quermittelachse des Inkontinenzartikels herangezogen.

Bei der Bestimmung des Querabstands (G) wird wiederum die Breite der Cuffsockellinie und die Breite des Schrittelastifizierungsmittels miterfasst, so wie dies in der Figur 3d angedeutet ist. Durch diese Konzeption der Cuffhöhe wird in bevorzugter Weise erreicht, dass die Schrittelastifizierungsmittel nicht nur einen physikalischen Seitenauslaufschutz bewirken, indem sie die Seitenrandbereiche des Schrittabschnitts gegen die Körperoberfläche, also gegen die Oberschenkel des Benutzers, anlegen, sondern zusätzlich eine in Querrichtung nach außen wirkende Zugkraft auf die benachbarte Cuffsockellinie ausüben, welche dann auch unterstützt durch die bootförmige Krümmung des Schrittabschnitts ein Aufstehen oder Emporerheben der Cuffelemente in Richtung auf die Körperoberfläche begünstigen.

Weiter erweist es sich insbesondere als vorteilhaft, wenn die Bemessung der Cuffhöhe an die Dicke des Absorptionskörpers gekoppelt ist. Hierfür wird vorgeschlagen, dass eine Quererstreckung (H) des von der körperzugewandten Seite des Artikels emporerhebbaren Bereichs des jeweiligen Cuffelements, also die Cuffhöhe, höchstens das 5,0-fache, insbesondere höchstens das 4,5-fache, insbesondere höchstens das 4,0-fache und wenigstens das 1,0-fache, insbesondere wenigstens das 1,5-fache, weiter insbesondere wenigstens das 2,0-fache einer Dicke des Absorptionskörpers beträgt, wobei die Quererstreckung (H) an einer in Querrichtung (16) schmalsten Stelle des Schrittabschnitts (8) bestimmt wird, die in der Längsrichtung (9) zwischen dem Bauchabschnitt (4) und dem Rückenabschnitt (6) liegt. Falls keine schmalste Stelle des Schrittabschnitts vorhanden ist, wie im Falle von geraden parallel verlaufenden Längsrändern, dann wird die Quermittelachse des Inkontinenzartikels herangezogen. Diese Dicke des Absorptionskörpers wird unter einem Prüfdruck von 20g/cm² und einer Prüffläche von 50 mm x 100 mm bestimmt.

Die Mitte der Entnahmeposition des Prüflings wird bestimmt durch den Kreuzungspunkt der Längsmittelachse des Inkontinenzartikels bzw. des Schrittabschnitts mit einer Querachse, die durch die schmalste Stelle des Schrittabschnitts verläuft. Falls keine schmalste Stelle des Schrittabschnitts vorhanden ist, entspricht diese Querachse der Quermittelachse des Inkontinenzartikels.

Die größere Längserstreckung des Prüflings soll der Richtung der Längsmittelachse des Inkontinenzartikels bzw. des Schrittabschnitts folgen. Im Falle von kleineren Abmessungen des Produkts ist auch die Entnahme von kleineren Prüflingen möglich. Hierfür muss dann die Kraft der Prüfvorrichtung entsprechend angepasst werden, damit ein Prüfdruck von 20 g/cm² bezogen auf die Prüffläche des Prüflings verbleibt.

Zur Bestimmung der Dicke wird der Prüfling durch die gesamte Dicke des Schrittabschnitts ausgestanzt. Es werden n = 5 Prüfungen durchgeführt; die Prüflinge werden in einer Dickenprüfanordnung gegenüber einem Prüfstempel von mindestens 100 x 100 mm zentriert angeordnet und mit einem Prüfdruck von 20 g/cm² belastet, die Verweildauer unter Belastung beträgt 5 ± 1 sec; die Höhe der Prüflinge wird dann an der Messskala abgelesen.

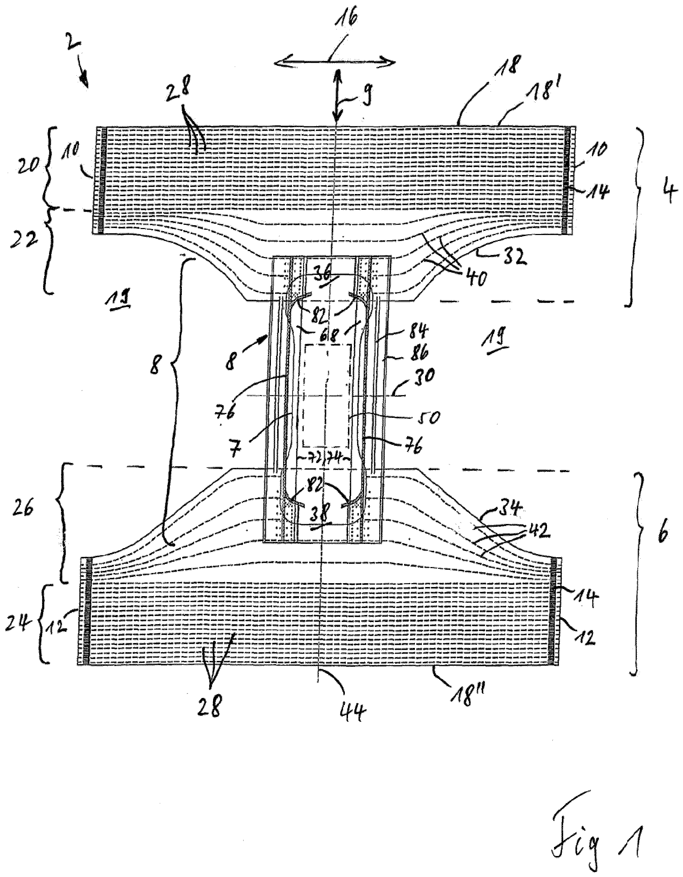

Diese Prüffläche ist in Form eines gestrichelten Rechtecks 50 in Figur 1 angedeutet, das bei der in Figur 1 beispielweise dargestellten bevorzugten Ausführung eines Schrittabschnitts mit geraden Längsrändern bezüglich der Längsmittelachse 44 und der Quermittelachse 30 zentriert ist.

Vorzugsweise weist der Absorptionskörper, gemessen nach beschriebener Methode eine Dicke von 5 - 12 mm, vorzugsweise 5 - 10 mm, weiter vorzugsweise von 5 - 8 mm auf.

Es erweist sich insbesondere weiter als vorteilhaft, wenn die aufeinander gefalteten Bereiche des Vliesmaterialabschnitts des jeweiligen Cuffelements in dem gesamten von der körperzugewandten Seite des Artikels emporerhebbaren Bereich nur lose gegeneinander anliegen, also frei von Fügestellen sind, d.h. nicht durch Fügestellen, insbesondere Klebe- oder Schweißstellen, insbesondere Ultraschallschweißstellen, miteinander verbunden sind. Somit werden die Cuffelemente als weich und angenehm empfunden. Und die Cuffelemente können sich flexibler der im Anwendungszustand entstandenen Umgebung anpassen.

Es erweist sich insbesondere weiter als vorteilhaft, wenn der emporerhebbare Bereich des jeweiligen Cuffelements nicht über Fügestellen mit in Querrichtung außerhalb der Cuffsockellinie liegenden Chassismaterialien oder Komponenten des Schrittabschnitts verbunden ist. Dies würde ebenfalls zu einer Versteifung der Cuffelemente führen, was im Lichte der vorliegenden Erfindung und der zugrundeliegenden Zielsetzung aber nicht beabsichtigt ist, sondern eher vermieden werden soll. Ferner würde dies zu einer Zwangsbeeinflussung im Sinne einer Zwangskopplung zu dem emporerhebbaren Bereich des Cuffelements führen, was den Tragkomfort wiederum beeinträchtigen könnte. Es wurde festgestellt, dass der mittelbare Einfluss der in Querrichtung außerhalb gelegenen Schrittelastifizierungsmittel zusammen mit der sich einstellenden Bootform des Schrittabschnitts hinreichend ist, um die Cuffelemente bzw. deren emporerhebbaren Bereich von der körperzugewandten Oberseite des Schrittabschnitts abzuheben.

Es wäre denkbar, dass die Cuffsockellinie entlang ihrer Erstreckung in Längsrichtung durchgehend gerade verläuft oder gerade und gekrümmte Abschnitte umfasst. Es erweist sich insbesondere als vorteilhaft, wenn der Inkontinenzartikel diesbezüglich so ausgebildet ist, dass die jeweilige Cuffsockellinie hinten und/oder vorn einen in Querrichtung nach innen, insbesondere bogenförmig nach innen, also in Richtung auf den unbefestigten freien Längsrand verlaufenden Cuffsockellinienabschnitt aufweist, welcher den emporerhebbaren Bereich des Cuffelements in der Längsrichtung begrenzt. Hierdurch wird bei dem jeweiligen Cuffelement eine Tasche gebildet, also das Cuffelement nach hinten bzw. nach vorn abgeschlossen, so dass das Wandern von Ausscheidungen in Richtung Rückenabschnitt bzw. Bauchabschnitt minimiert werden kann. Weiter trägt ein in Querrichtung, insbesondere bogenförmig verlaufender Cuffsockellinienabschnitt vorteilhaft zu einem planen Festlegen der Cuffelemente bei, was den Komfort für den Anwender unterstützt. Weiter kann mit einem solchen in Querrichtung, insbesondere bogenförmigen verlaufenden Cuffsockellinienabschnitt den beim Anlegevorgang und Positionieren des Inkontinenzartikels am Anwender vorgenommenen Bewegungen in Querrichtung- bzw. Hüftumgangsrichtung und den damit einhergehenden Zug- und Reibungskräften auf das Cuffelement und des emporerhebbaren Bereich gut begegnet werden, da eine in Querrichtung verlaufende Fixierung des emporerhebbaren Bereichs des Cuffelements solchen Kräften weniger ausgesetzt ist.

Bei einer bevorzugten Ausführungsform, bei der die jeweilige Cuffsockellinie hinten und vorn einen in Querrichtung,insbesondere bogenförmig nach innen verlaufenden Cuffsockellinienabschnitt aufweist, kann es sich insbesondere als vorteilhaft erweisen, dass die Cuffsockellinie in dem Bereich zwischen den beiden in Querrichtung, insbesondere bogenförmig nach innen verlaufenden Cuffsockellinienabschnitten gerade verläuft.

Weiter kann es sich insbesondere als vorteilhaft erweisen, dass die jeweilige Cuffsockellinie über ihre im Wesentlichen gesamte Längserstreckung gerade erstreckt ist, und hiervon ausgehend hinten und/oder vorn einen zusätzlichen in Querrichtung, insbesondere bogenförmig nach innen, also in Richtung auf den unbefestigten freien Längsrand verlaufenden Cuffsockellinienabschnitt aufweist oder aufweisen kann, welcher den emporerhebbaren Bereich des Cuffelements in der Längsrichtung begrenzt. Eine solche Ausführung trägt vorteilhaft zu einer verstärkten Fixierung der Cuffelemente und damit zu deren Stabilität bei, da solchenfalls einem Kräfteeintrag durch Zug- und Reibungskräfte auf die Cuffelemente sowohl in Längs- als auch in Querrichtung begegnet werden kann.

Die Cuffsockellinie des jeweiligen Cuffelements kann außerhalb und/oder oberhalb des Absorptionskörpers verlaufen. Es kann sich insbesondere als vorteilhaft erweisen, dass die jeweilige Cuffsockellinie innerhalb eines in der Längsrichtung mittleren Bereichs des Schrittabschnitts außerhalb des Absorptionskörpers, also neben Längsrändern des Absorptionskörpers verläuft und in einem vorderen und/oder hinteren Bereich des Schrittabschnitts auf dem Absorptionskörper verläuft. Eine solche Kombination der Positionierung der Cuffsockellinie kann auch positiv zum Emporerheben der Cuffelemente beitragen, da das durch eine erhöhte Platzierung der Cuffsockellinie auf dem Absorptionskörper bewirkte Gefälle zu einer gewissen Reckung des in Längsrichtung mittleren Bereichs des emporerhebbaren Bereichs führt.

Vorzugsweise weist der Absorptionskörper eine konturierte Form auf, mit zwischen dessen Endbereichen vorhandenem mittleren Bereich von geringerer Quererstreckung.

Vorzugsweise weisen die freien Längsränder der Cuffelemente einen Querabstand E, wie in Figur 3d dargestellt, voneinander von 60 - 90 mm, vorzugsweise von 65-85 mm, vorzugsweise von 65 - 80 mm auf.

Vorzugsweise sind die Cuffsockellinien in einem Querabstand D, wie in Figur 3d dargestellt, von 90 - 130 mm, insbesondere von 90 - 120 mm, weiter insbesondere von 95 - 115 mm voneinander beabstandet. Im Falle von vom emporerhebbaren Bereich des Cuffelements einwärts verlaufenden Cuffsockellinien, so werden diese in die Abstandsmessung miteinbezogen.

Im Hinblick auf das eingangs genannte Ziel der Schaffung eines diskret tragbaren Produkts ist eine Längserstreckung des Schrittabschnitts vorzugsweise kürzer als eine Gesamtlängserstreckung des Inkontinenzartikels. Somit verbleiben im Bauchabschnitt und/oder Rückenabschnitt Bereiche in der Längsrichtung, in denen keine Überlappungs mit dem Schrittabschnitt vorgesehen ist, was auch das Tragegefühl positiv unterstützt.

Insbesondere erweist es sich als vorteilhaft, dass eine Längserstreckung des Schrittabschnitts wenigstens das 0,45-fache, insbesondere wenigstens das 0,50-fache und höchstens das 0,70-fache, insbesondere höchstens das 0,65-fache, insbesondere höchstens das 0,60-fache einer Gesamtlängserstreckung des Inkontinenzartikels beträgt.

Es erweist sich weiter insbesondere als vorteilhaft, dass ein vorderes und/oder hinteres Ende des Schrittabschnitts und jedenfalls ein jeweiliges vorderes und/oder hinteres Längsende des Absorptionskörpers des Schrittabschnitts in der Längsrichtung vor einem schrittseitigen Ende der Seitennahtbereiche endet. Eine solche Anordnung des Schrittabschnitts und Absorptionskörpers unterstützt vorteilhaft die Ausführungsform eines diskreten Inkontinenzartikels, da Versteifung, Verwindung und damit womöglich im Nutzungszustand durch die Kleidung erkennbare Abdrücke erzeugende Bereiche weitgehend im Schrittbereich verbleiben.

Es erweist sich weiter insbesondere als vorteilhaft, dass eine Fläche des Schrittabschnitts im eben ausgebreiteten Zustand 12 - 30 %, insbesondere 15 - 25 %, insbesondere 15 - 23 % einer Produktgesamtfläche des Inkontinenzartikels beträgt.

Es erweist sich insbesondere weiter als vorteilhaft, dass eine Fläche des Absorptionskörpers im eben ausgebreiteten Zustand 7 - 15 %, insbesondere 8 - 13 % einer Produktgesamtfläche des Inkontinenzartikels beträgt.

Es erweist sich insbesondere weiter als vorteilhaft, dass eine Fläche des Schrittabschnitts im eben ausgebreiteten Zustand 60.000 - 80.000 mm², insbesondere 60.000 - 75.000 mm², weiter insbesondere 62.000 - 72.000 mm² und/oder eine Fläche des Absorptionskörpers im eben ausgebreiteten Zustand 30.000 - 45.000 mm², insbesondere 30.000 - 42.000 mm², insbesondere 32.000 - 40.000 mm² beträgt.

Der Schrittabschnitt weist vorzugsweise eine Längserstreckung von 300 - 500 mm, insbesondere von 350 - 450 mm auf.

Eine maximale Erstreckung des Schrittabschnitts in Querrichtung beträgt vorzugsweise 100 - 250 mm, insbesondere 120 - 230 mm, weiter insbesondere 140 - 200 mm.

Der Schrittabschnitt weist vorzugsweise gerade Längsseitenkanten auf.

Der Absorptionskörper weist vorzugsweise eine Längserstreckung von 200 - 450 mm, insbesondere von 250 - 420 mm, weiter insbesondere von 280 - 400 mm auf und/oder eine Quererstreckung, also Breite K, vorzugsweise eine maximale Quererstreckung von 70- 160 mm, insbesondere von 85 - 145 mm, weiter insbesondere von 100 - 130 mm auf. Insbesonders vorteilhaft ist eine konturierte Ausbildung des Absorptionskörpers mit einem vorzugsweise zwischen dessen Endbereichen vorhandenem mittleren Bereich von geringerer Quererstreckung.

Es erweist sich insbesondere weiter als vorteilhaft, dass der Schrittabschnitt 4 - 15 %, insbesondere 4 - 12 %, insbesondere 4 - 10 % der Fläche des Bauchabschnitts überlappt und/oder dass der Schrittabschnitt 5 - 20 %, insbesondere 5 - 16 %, insbesondere 5 - 14 %, weiter insbesondere 5 - 12 % der Fläche des Rückenabschnitts überlappt.

Wiederum im Hinblick auf die Schaffung eines schmalen Produkts erweist es sich insbesondere als vorteilhaft, dass eine maximale Quererstreckung des Schrittabschnitts wenigstens das 0,15-fache, insbesondere wenigstens das 0,18-fache, insbesondere wenigstens das 0,20-fache und höchstens das 0,30-fache, insbesondere höchstens das 0,28-fache einer maximalen Quererstreckung des Inkontinenzartikels beträgt.

Bei dem hier in Rede stehenden dreiteiligen Aufbau des Inkontinenzartikels erweist es sich insbesondere als vorteilhaft, wenn der Schrittabschnitt gewissermaßen als unitäre Einheit, vorzugsweise mit eingebrachten und fixierten Cuffelementen und vorzugsweise einschließlich der Schrittelastifizierungsmittel ausgebildet und als solche bezüglich des Bauchabschnitts und des Rückenabschnitts in Überlappung positioniert und fixiert wird. Hierfür erweist es sich insbesondere als vorteilhaft, dass der Schrittabschnitt ein flüssigkeitsundurchlässiges Backsheet-Material und ein flüssigkeitsdurchlässiges Topsheet-Material umfasst, zwischen denen der Absorptionskörper angeordnet ist. Hierbei können das Backsheet-Material und/oder das Topsheet-Material in Querrichtung einen Überhang über den Absorptionskörper bilden. Solchenfalls erweist es sich als vorteilhaft, wenn die Schrittelastifizierungsmittel zumindest mit dem Topsheet-Material oder mit dem Backsheet-Material oder mit einem in Querrichtung nach außen verlaufenden Bereich des Vliesmaterialabschnitts, welcher das Cuffelement bildet, verbunden ist.

Der Schrittabschnitt umfasst vorteilhafterweise ein flüssigkeitsundurchlässiges Backsheet-Material und ein Vlies-Topsheet-Material. Das Backsheet umfasst insbesondere eine Folie, insbesondere eines Flächengewichts von 8-25 g/m².

Das Topsheet-Material umfasst insbesondere ein Vliesmaterial, insbesondere eines Flächengewichtes von 6-20 g/m², insbesondere von 8 - 18 g/m², weiter insbesondere von 10 - 15 g/m².

Als Schrittelastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra^{®}- oder Spandex^{®}-Fäden eingesetzt. Die Schrittelastifizierungsmittel haben vorzugsweise eine Stärke von 300-1500 dtex, insbesondere von 500-1200 dtex, weiter insbesondere von 500-900 dtex.

Die Schrittelastifizierungsmittel werden vorzugsweise unter einer Vorspannung von 1,5-6,0, insbesondere von 2,5-4,5 an den chassisbildenden Hüllmaterialien des Schrittabschnitts, wie Backsheet, Topsheet und/oder einem in Querrichtung nach außen verlaufenden Bereich des Vliesmaterialabschnitts fixiert.

Die Vorspannung ist definiert als Dehnungsgrad eines gedehnten Elastifizierungsmittels gegenüber dem ungedehnten/relaxierten Ausgangszustand des Elastifizierungsmittels im Zustand des Aufbringens und Fixierens des Elastifizierungsmittels in der Herstellungsmaschine. Der Dehnungsgrad errechnet sich also als Verhältnis der gedehnten Länge L' (= Ausgangslänge L + ΔL) zur Ausgangslänge L, also L'/L.

Weiter erweist es sich als vorteilhaft, dass eine Längserstreckung der jeweiligen Seitennahtbereiche das 0,18 - 0,30-fache, insbesondere 0,18 - 0,28-fache, insbesondere das 0,18 - 0,25-fache einer maximalen Quererstreckung des Inkontinenzartikels beträgt.

Die für einen Inkontinenzartikel in Höschenform erforderliche elastische Dehnbarkeit in Quer- oder Hüftumfangsrichtung kann in vorteilhafter Weise dadurch realisiert werden, dass in dem Bauchabschnitt und/oder in dem Rückenabschnitt erste Elastifizierungsmittel vorgesehen sind, die sich in einem Abstand voneinander und im Wesentlichen in Quer- oder Hüftumfangsrichtung erstrecken und so den Bauchabschnitt und/oder den Rückenabschnitt flächenhaft elastifizieren, oder dass der Bauchabschnitt und/oder der Rückenabschnitt in Quer- oder Hüftumfangsrichtung elastisch dehnbare Flächenmaterialien umfasst. Eine Querelastifizierung ist also vom Grundsatz her dadurch möglich, dass im Wesentlichen fadenförmige Elastifizierungsmittel eingesetzt werden oder dass streifenförmige Elastifizierungsmittel oder alternativ auch zumindest in Querrichtung elastisch dehnbare Flächenmaterialien eingesetzt werden. In jedem Fall erweist es sich als vorteilhaft, wenn elastisch dehnbare Materialien mit im Wesentlichen undehnbaren Materialien, wie insbesondere Vliesmaterialien, kombiniert werden.

Der Inkontinenzartikel in Höschenform ist insbesondere ein Erwachsenen-Inkontinenzartikel. Insbesondere ist der Inkontinenzartikel für die Verwendung durch Frauen vorgesehen, er ist somit insbesondere ein Frauen-Inkontinenzartikel.-

Gegenstand der Erfindung ist auch eine Anordnung (Array) aus einem ersten und einem zweiten Inkontinenzartikel, die einander im Hinblick auf verschiedene Benutzungssituationen in vorteilhafter Weise ergänzen und die sich in einer oder in mehrfacher Hinsicht voneinander unterscheiden.

Die Anordnung (Array) ergibt sich dabei durch die sinnfällige Herrichtung der zum Array gehörenden Inkontinenzartikel. Insbesondere durch die Beziehung oder das Verhältnis der Artikel zueinander. Diese erfolgt entweder durch Darbietung in einer gemeinsamen Verpackungseinheit und/oder bevorzugt durch die Anbringung von Kennzeichnungen auf den Inkontinenzartikeln und/oder deren Verpackung und/oder die Darbietung in räumlicher oder inhaltlicher Nähe zueinander, die die Zugehörigkeit zu einem Array kenntlich machen. Die den Array bildenden Artikel stammen bevorzugt von ein und demselben Hersteller.

Die einen Array bildenden Inkontinenzartikel weisen bevorzugt dieselbe Produktkennzeichnung auf, wie eben Markennamen und/oder Sub-Markennamen.

Eine Anordnung aus einem ersten Inkontinenzartikel und einem zweiten Inkontinenzartikel wird verstanden als zumindest jeweils einem Vertreter davon und schließt eine Mehrzahl des einen ersten und/oder des einen zweiten Inkontinenzartikels ein.

Erfindungsgemäß wird vorliegend daher eine Anordnung (Array) aus einem ersten Inkontinenzartikel mit den Merkmalen nach einem oder mehreren der Ansprüche 1 bis 20 und einem zweiten Inkontinenzartikel mit den im Anspruch 21 genannten Merkmalen beansprucht. Die beiden Artikel unterscheiden sich also hinsichtlich der nichtelastischen bzw. elastischen Ausbildung des emporerhebbaren Bereichs der Cuffelemente.

Insbesondere bevorzugt unterscheiden sich der erste und zweite Inkontinenzartikel weiter in den Abmessungen des Schrittabschnitts und des Absorptionskörpers. Dabei bildet der Artikel mit kleinerem Schrittabschnitt und kleinerem Absorptionskörper den ersten Inkontinenzartikel mit nichtelastifiziertem Cuffelement und der Artikel mit größerem Schrittabschnitt und größeren Absorptionskörper den zweiten Inkontinenzartikel mit zumindest teilweise elastifiziertem Cuffelement. Es hat sich nämlich gezeigt, dass gerade bei einer Verwendung des betreffenden Inkontinenzartikels durch Frauen eine Nichtelastifizierung der Cuffelemente bei kleinen schmalbauenden Schrittabschnitten und Absorptionskörpern als angenehmer empfunden wird, während bei demgegenüber ausladender bauenden Artikeln eine längselastische Ausbildung des emporerhebbaren Bereichs der Cuffelemente als weniger störend empfunden wird und funktional wirksam sein kann.

Vorzugsweise ist die Anordnung derart, dass für den Anwender die unterschiedlichen Benutzungszustände von erstem und zweitem Inkontinenzartikel erkennbar sind.

Insbesondere ist der erste Inkontinenzartikel für die Anwendung bei leichterer Inkontinenz und der zweite Inkontinenzartikel für die Anwendung für einen davon verschiedenen stärkeren Grad an Inkontinenz vorgesehen.

Insbesondere umfasst die Anordnung zumindest einen ersten und zumindest einen zweiten Inkontinenzartikel, die für die Verwendung durch Frauen vorgesehen sind, zumal der erste Inkontinenzartikel mit Cuffelementen ohne Elastifizierung den anatomischen Gegegebenheiten des weiblichen Körpers besonders vorteilhaft gerecht wird.

Im Vergleich von zumindest einem ersten Inkontinenzartikel und zumindest einem zweiten Inkontinenzartikel wird insbesondere auf Inkontinenzartikel von im wesentlichen gleicher Größe abgestellt. Größe wird dabei verstanden, was im fachüblichen Gebrauch im technischen Feld der Inkontinenzartikel mit den Größenbezeichnungen wie beispielsweise XS, S, M, L, XL zugeordnet ist. Ein erster Inkontinenzartikel z.B. der Größe M und ein zweiter Inkontinenzartikel der Größe M weisen dabei eine im Wesentlichen gleiche maximale Querstreckung des Inkontinenzartikels auf. Als eine im Wesentlichen gleiche maximale Quererstreckung wird hierin verstanden, dass eine Abweichung der Produktquererstreckung des ersten und zweiten Inkontinenzartikels um höchstens 10%, insbesondere höchstens 8%, weiter insbesondere höchstens 6% voneinander zulässig ist.

Dabei können sich der erste und zweite Inkontinenzartikel bei im wesentlichen gleicher Größe im vorstehend genannten Sinn dennoch in ihrer Produktlängserstreckung unterscheiden, wobei der erste Inkontinenzartikel vorzugsweise kürzer ist.

Weiter vorzugsweise unterscheiden sich der erste und zweite Inkontinenzartikel in der Querstreckung des von der körperzugewandten Seite des Artikels emporerhebbaren Bereichs des jeweiligen Cuffelements, also in der Cuffhöhe, wobei die Cuffhöhe des ersten Inkontinenzartikels vorzugsweise geringer ist.

Weiter vorzugsweise unterscheiden sich der erste und zweite Inkontinenzartikel in der Querstreckung des von der körperzugewandten Seite des Artikels emporerhebbaren Bereichs des jeweiligen Cuffelements, also in der Cuffhöhe in Bezug auf die Quererstreckung F des Schrittabschnitts ab Cuffsockellinie (H/F) und/oder in Bezug auf den Querabstand G zwischen Cuffsockellinie und eines äußersten Schrittelastifizierungsmittels (H/G). Vorzugsweise weist der erste Inkontinenzartikel ein gegenüber dem zweiten Inkontinenzartikel im Betrag kleineres Verhältnis H/F auf und/oder ein gegenüber dem zweiten Inkontinenzartikel im Betrag kleineres Verhältnis H/G auf.

Die nachfolgenden Merkmalsaufzählungen können sich in beliebiger Kombination mit vorstehend erläuterten Merkmalen eines Inkontinenzartikels, insbesondere sowohl für einen ersten als auch einen zweiten Inkontinenzartikel als vorteilhaft erweisen:
- dass der Bauchabschnitt und/oder der Rückenabschnitt vorzugsweise eine von der Quer- oder Hüftumfangsrichtung abweichende und in Richtung auf eine Quermittelachse des Schrittabschnitts zulaufende Randkontur zur Begrenzung der Beinöffnungen aufweisen.
- dass in einem schrittseitigen und den Beinöffnungen zugewandten Bereich des Bauchabschnitts und/oder des Rückenabschnitts vorzugsweise zweite Elastifizierungsmittel vorgesehen sind, die sich ausgehend von den beiden Seitennahtbereichen in Richtung auf eine Längsmittelachse des Inkontinenzartikels erstrecken und dabei bogenförmig sich auffächernd mit zunehmendem Abstand voneinander verlaufen.
- dass ein minimaler Abstand der zweiten Elastifizierungsmittel voneinander in den Seitennahtbereichen vorzugsweise 3 - 8 mm, insbesondere 3 - 7 mm und weiter insbesondere 3 - 6 mm beträgt.
- dass ein maximaler Abstand der zweiten Elastifizierungsmittel voneinander an einem Absorptionskörperrand oder an einem Längsrand des Schrittabschnitts vorzugsweise 10 - 35 mm, insbesondere 12 - 35 mm und weiter insbesondere 15 - 32 mm beträgt.
- dass der maximale Abstand der zweiten Elastifizierungsmittel voneinander an einem Absorptionskörperrand oder an einem Längsrand des Schrittabschnitts im Rückenabschnitt vorzugsweise größer ist als im Bauchabschnitt.
- dass die ersten und/oder zweiten

Elastifizierungsmittel vorzugsweise in einem Überlappungsbereich mit dem Absorptionskörper des Schrittabschnitts hinsichtlich ihrer elastischen Eigenschaften deaktiviert sind.
- dass der Abstand des schrittzugewandten innersten zweiten Elastifizierungsmittels des Bauchabschnitts von dem entsprechenden schrittzugewandten innersten zweiten Elastifizierungsmittel des Rückenabschnitts vorzugsweise 220 - 420 mm, insbesondere 220 - 400 mm, weiter insbesondere 220 - 350 mm beträgt.
- dass der Abstand des innersten, schrittzugewandten zweiten Elastifizierungsmittels von der die Beinöffnungen begrenzenden Randkontur des schrittseitigen und den Beinöffnungen zugewandten Bereichs des Bauchabschnitts und/oder des Rückenabschnitts vorzugsweise 2 - 40 mm, vorzugsweise 3 - 30 mm, insbesondere vorzugsweise 4 - 15 mm beträgt.

- dass die Erstreckung des Bauchabschnitts und des Rückenabschnitts im Seitennahtbereich in Längsrichtung vorzugsweise wenigstens 100 mm, insbesondere wenigstens 120 mm und insbesondere 120 mm - 220 mm, weiter insbesondere 120 -180 mm beträgt.
- dass der minimale Abstand des Bauchabschnitts und des Rückenabschnitts in Längsrichtung voneinander vorzugsweise 200 - 400 mm, weiter vorzugsweise 200 - 380 mm beträgt.
- dass der Schrittabschnitt vorzugsweise ein flüssigkeitsundurchlässiges Backsheet-Material und ein Topsheet-Material umfasst, zwischen denen der Absorptionskörper angeordnet ist, wobei das Backsheet-Material und/oder das Topsheet-Material in Querrichtung vorzugsweise einen Überhang über den Absorptionskörper bilden können und dieser Überhang - in der Summe beidseits des Absorptionskörpers, also in der Summe links und rechts, vorzugsweise wenigstens 18 %, insbesondere 20 - 35 % und weiter insbesondere 25 - 32 % bezogen auf die größte Breite, also Quererstreckung des Schrittabschnitts beträgt.
- dass die Schrittelastifizierungsmittel in Längsrichtung vorzugsweise vor den ersten und/oder zweiten Elastifizierungsmitteln enden.
- dass die Schrittelastifizierungsmittel in Längsrichtung vorzugsweise den Bauchabschnitt und/oder den Rückenabschnitt überlappen.
- dass der Bauchabschnitt und der Rückenabschnitt zumindest außerhalb des Absorptionskörpers über die Längsrichtung vorzugsweise im Wesentlichen durchgehend flächenhaft querelastifiziert sind.
- dass die Überlappung des Schrittabschnitts mit dem Rückenabschnitt vorzugsweise größer ist als mit dem Bauchabschnitt.

Weitere Einzelheiten, Merkmale und Vorteile der Erfindung ergeben sich aus den beigefügten Patentansprüchen und aus der zeichnerischen Darstellung und nachfolgenden Beschreibung bevorzugter Ausführungsformen des erfindungsgemäßen Inkontinenzartikels.

In der Zeichnung zeigt:
Figur 1 eine Draufsicht auf einen erfindungsgemäßen Inkontinenzartikel, wobei ein Bauchabschnitt, ein Rückenabschnitt und ein die beiden überbrückender und verbindender Schrittabschnitt des Inkontinenzartikels noch nicht zur Bildung einer Höschenform verbunden, sondern in flach gelegtem und eben ausgebreiteten Zustand dargestellt sind;
Figur 2 eine teilweise dargestellte perspektivische Ansicht des Schrittabschnitts im Bereich einer Quermittelachse;
Figur 3a eine Figur 1 entsprechende Draufsicht auf den Schrittabschnitt mit Schnittebenen und nur angedeutetem Bauchabschnitt und Rückenabschnitt;
Figur 3b eine schematische Schnittansicht des Schrittabschnitts mit Schnittebene A-A in Figur 3a;
Figur 3c eine schematische Schnittansicht des Schrittabschnitts mit Schnittebene B-B in Figur 3a;
Figur 3d die Schnittansicht nach Figur 3b mit eingezeichneten Abmessungen;
Figur 4 eine Draufsicht auf den Inkontinenzartikel nach Figuren 1-3 und einen weiteren Inkontinenzartikel, die ein Array bilden; und
Figuren 5a und 5b jeweils eine schematische Schnittansicht des Schrittabschnitts eines weiteren Inkontinenzartikels nach Figur 4.

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 mit einem Flächenanteil des Bauchabschnitts 4 einerseits und des Rückenabschnitts 6 andererseits überlappt und im Überlappungsbereich 36, 38 herstellerseitig unlösbar verbunden ist. Wie aus Figur 1 ersichtlich, führt dies zu einer H-förmigen Grundstruktur des Inkontinenzartikels mit einer Längsrichtung 9. Die in Figur 1 dargestellten miteinander gefügten Bestandteile werden dann zur Bildung der Höschenform an jeweiligen seitlichen Längsrandabschnitten 10, 12 des Bauchabschnitts 4 und des Rückenabschnitts 6 ebenfalls herstellerseitig miteinander verbunden, wodurch beidseits Seitennahtbereiche 14 ausgebildet werden. In diesem herstellerseitig gefertigten höschenförmigen Zustand des Inkontinenzartikels erstrecken sich der Bauchabschnitt 4 und der Rückenabschnitt 6 in Quer- oder Hüftumfangsrichtung 16 durchgehend bis zu den Seitennahtbereichen 14 und definieren so eine in Hüftumfangsrichtung geschlossene Hüftöffnung 18 mit einer vorderen 18' und einer hinteren 18" Hüftöffnungskante und Beinöffnungen 19, durch welche hindurch der Benutzer den Inkontinenzartikel wie ein Höschen anlegt.

Der Bauchabschnitt 4 lässt sich bei der beispielhaften Ausführungsform in einen hüftseitigen Bereich 20 und in einen schrittseitigen und den Beinöffnungen zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen zugewandten Bereich 26.

In dem hüftseitigen Bereich 20 des Bauchabschnitts 4 und in dem hüftseitigen Bereich 24 des Rückenabschnitts 6 sind erste Elastifizierungsmittel 28 vorgesehen, bei denen es sich insbesondere um fadenförmige Elastifizierungsmittel, wie Lycra^{®}-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28 erstrecken sich in Quer- oder Hüftumfangsrichtung 16 von einem Seitennahtbereich 14 zum anderen.

Der schrittseitige und den Beinöffnungen 19 zugewandte Abschnitt 22 des Bauchabschnitts 4 bzw. 26 des Rückenabschnitts 6 haben eine von der Quer- oder Hüftumfangsrichtung 16 abweichende in Richtung auf eine Quermittelachse 30 des Inkontinenzartikels zulaufende Randkontur 32 bzw. 34. Diese Randkontur 32, 34 ist abschnittsweise bogenförmig und daher zur Begrenzung der Beinöffnungen 19 geeignet. Durch diesen Verlauf des schrittseitigen und den Beinöffnungen zugewandten Bereichs 22 bzw. 26 wird auch ein Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist.

Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus einer bevorzugten Ausführungsform in den Figuren zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt.

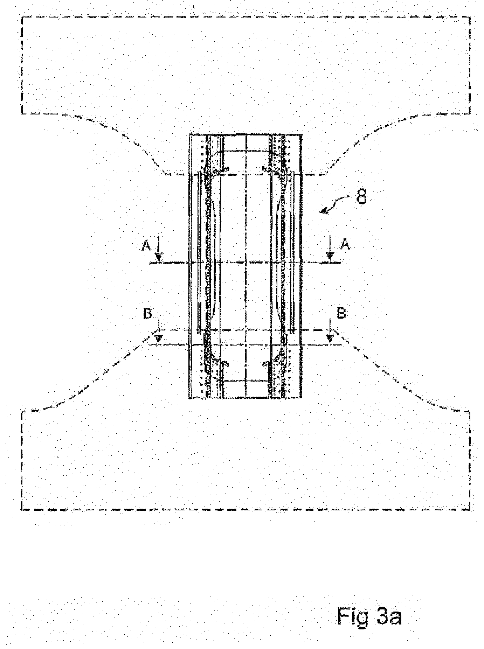

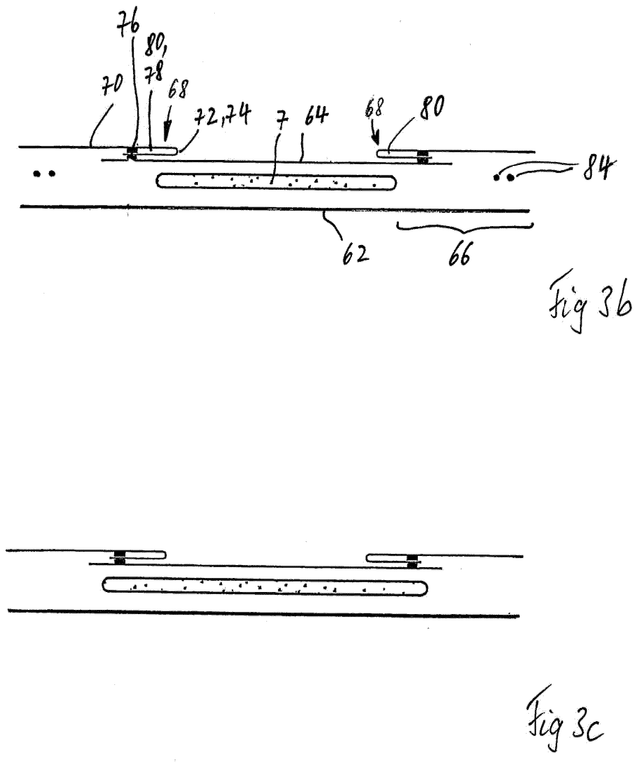

Der Schrittabschnitt 8 umfasst ein flüssigkeitsundurchlässiges Backsheet 62, das insbesondere von einem atmungsaktiven, jedoch flüssigkeitsdichten Folienmaterial gebildet sein kann, und ein, vorzugsweise auf Vliesbasis beruhendes flüssigkeitsdurchlässiges Topsheet 64. Zwischen dem Backsheet 62 und dem Topsheet 64 ist wie aus den Figuren 3 ersichtlich der Absorptionskörper 7 angeordnet. Im beispielhaft dargestellten Fall bildet das Backsheet 62 in Querrichtung 16 einen Überhang 66 über den Absorptionskörper 7. Das Topsheet 64 überragt den Absorptionskörper 7 in Querrichtung nur verhältnismäßig geringfügig; jedoch ist beidseits und entlang des Absorptionskörpers 7 in Längsrichtung 9 verlaufend jeweils ein aufstehendes Barrieremittel vorgesehen, welches als aufstehendes Cuffelement 68 bezeichnet wird und vorzugsweise aus einem hydrophoben, insbesondere flüssigkeitsundurchlässigen Vliesstoffmaterial gebildet ist.

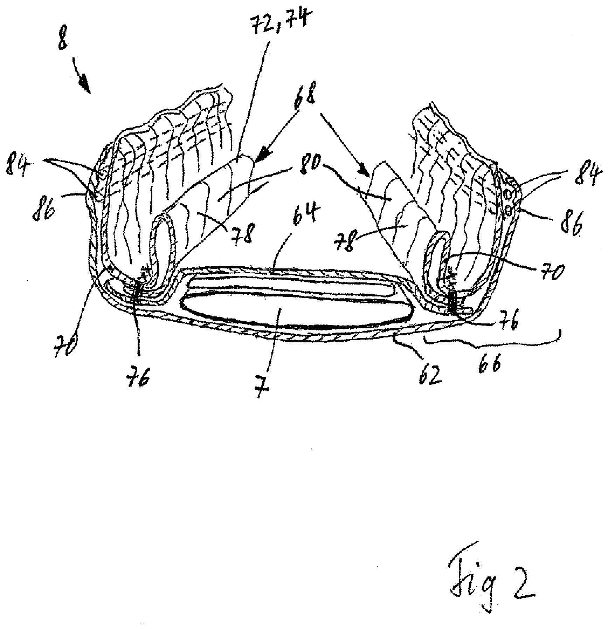

Das Cuffelement 68 ist aus einem auf sich selbst entlang der Längsrichtung 9 umgefalteten Vliesmaterialabschnitt 70 gebildet, wobei eine hierbei entstehende Faltlinie 72 einen unbefestigten freien Längsrand 74 bildet, der aber wie in Figur 2 gezeigt verrundet ausgebildet ist.

Der auf sich selbst umgefaltete Vliesmaterialabschnitt 70 ist auf die körperzugewandte Seite von Topsheet und/oder Backsheet aufgebracht und dort entlang einer sogenannten Cuffsockellinie 76 mit dem Topsheet 64 oder Backsheet 62 gefügt. Die Cuffsockellinie 76 verläuft über ihre gesamte Längserstreckung ganz in dem umgefalteten Bereich 78 des Vliesmaterialabschnitts 70. Hierdurch wird also auch dieser umgefaltete Bereich 78 des Vliesmaterialabschnitts 70 durch die Cuffsockellinie 76 selbst fixiert. Weiter wird hierdurch in Querrichtung innerhalb dieser Cuffsockellinie 76 ein emporerhebbarer Bereich 80 des Cuffelements 68 begrenzt und definiert. Dieser emporerhebbare Bereich 80 des Cuffelements 68 weist außer der Cuffsockellinie 76 auch keine weiteren Fügeverbindungen der aufeinander gefalteten Vliesmaterialien auf; diese liegen vielmehr lose gegeneinander an, was ein angenehmes Traggefühl vermittelt. Der emporerhebbare Bereich 80 ist also nicht über weitere Fügeverbindungen außer der Cuffsockellinie 76 angebunden; er ist somit nicht mit weiteren Komponenten zwangsgekoppelt, was eine ungewollte Versteifung bewirken könnte.

Im beispielhaft bevorzugt dargestellten Fall, wie in Figur 1 dargestellt, erstreckt sich die Cuffsockellinie 76 im Wesentlichen über die gesamte Längserstreckung des Cuffelements 68 in einer geraden Linie. Es ist jedoch hinten und vorn ausgehend von der geraden Erstreckung ein zusätzlicher in Querrichtung 16 bogenförmig nach innen, also in Richtung auf den unbefestigten freien Längsrand 74 verlaufender Cuffsockellinienabschnitt 82 ausgebildet, der den emporerhebbaren Bereich 80 des Cuffelements 68 nach hinten und nach vorn begrenzt. Es wird auf diese Weise eine Art Tasche gebildet.

In Querrichtung außerhalb und beidseits des Absorptionskörpers 7 sind in der Längsrichtung 9 verlaufende und der jeweiligen Beinöffnung 19 zugeordnete Schrittelastifizierungsmittel 84 vorgesehen. Sie sind im beispielhaft dargestellten Fall mit einem Querabstand zur Cuffsockellinie zwischen dem Vliesmaterialabschnitt 70 des Cuffelements 68 und dem Backsheet-Material 62 angeordnet. Sie vermögen somit den Schrittabschnitt 8 in der Längsrichtung 9 zu elastifizieren und eine Raffung der Chassismaterialien des Schrittabschnitts 8 herbeizuführen. In der Gebrauchssituation, die in Figur 2 schematisch dargestellt ist, erheben sich daher die Längsrandbereiche 86 des Schrittabschnitts 8 gegen die Beine des Benutzers und bilden einen Seitenauslaufschutz des Inkontinenzartikels. Durch die hiermit verbundene Bootform des Schrittabschnitts 8 erheben sich auch die emporerhebbaren Bereiche 80 der jeweiligen Cuffelemente 68, was in Figur 2 schematisch dargestellt ist.

Die Figuren 3a bis d zeigen eine Draufsicht bzw. zwei Schnittansichten (jeweils schematisch) des Schrittabschnitts 8 im eben ausgebreiteten Zustand. Figur 3d zeigt und verdeutlicht folgende Bemessungen:
- Breite K des Absorptionskörpers 7;
- Querabstand E der freien Längsränder 74 der Cuffelemente 68 voneinander;
- Querabstand D der Cuffsockellinien 76 voneinander;
- Quererstreckung H des emporerhebbaren Bereichs 80 des jeweiligen Cuffelements 68;
- Querabstand G zwischen der Cuffsockellinie 76 (inklusive Breite der Cuffsockellinie) und einem in Querrichtung äußersten Schrittelastifizierungsmittel 84 (inklusive Breite des Schrittelastifizierungsmittels);
- Quererstreckung F des Schrittabschnitts gemessen von der Cuffsockellinie 76 (inklusive Breite der Cuffsockellinie) in Querrichtung nach außen.

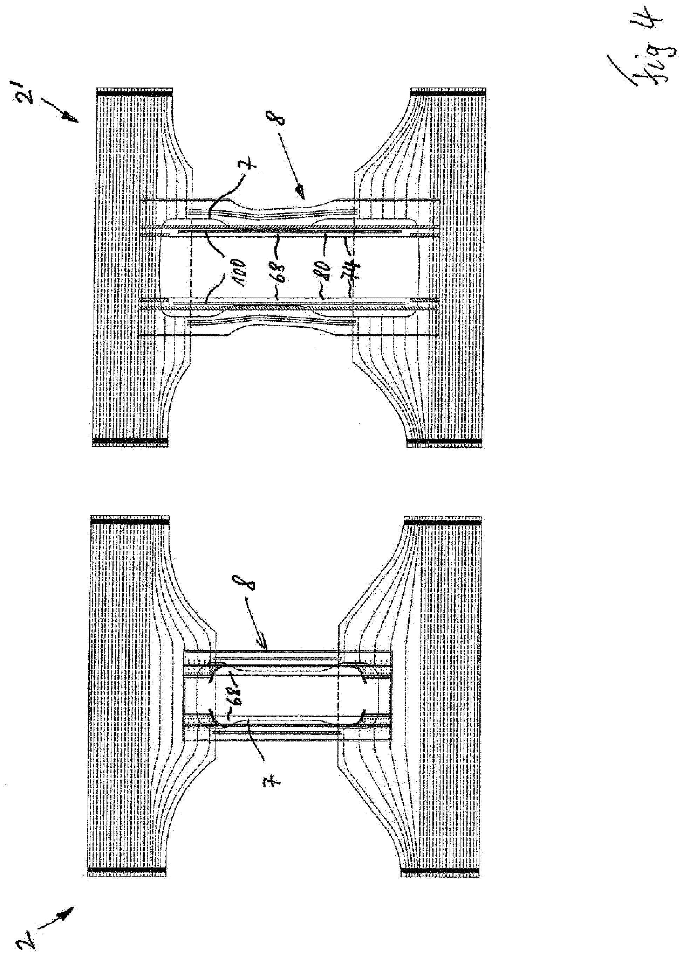

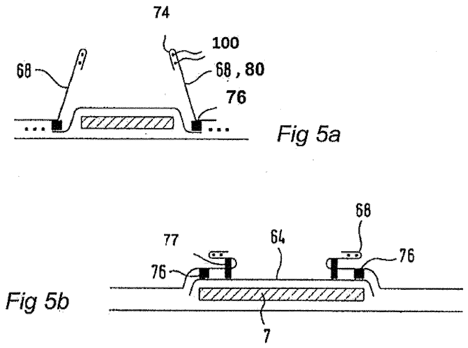

Die Figur 4 zeigt eine Draufsicht auf einen weiteren zweiten Inkontinenzartikel 2', der sich von dem bislang beschriebenen Inkontinenzartikel 2, der ebenfalls dargestellt ist, insbesondere dadurch unterscheidet, dass sein Schrittabschnitt 8 und sein Absorptionskörper 7 länger und breiter ausgebildet sind und ferner dadurch, dass ein emporerhebbarer Bereich 80 des Cuffelements 68, insbesondere entlang seines unbefestigten freien Längsrands 74, in der Längsrichtung 9 elastisch oder elastifiziert ausgebildet ist. Das jeweilige Cuffelement 68 umfasst dort in der Längsrichtung erstreckte Elastifizierungsmittel 100, insbesondere fadenförmige Elastifizierungsmittel 100, wobei auch andere Formen der Längselastifizierung denkbar sind. Figur 5a zeigt beispielhaft eine solche Ausgestaltung der Cuffelemente des zweiten Inkontinenzartikel 2' in einer Schnittansicht des Schrittabschnitts. Entlang einer Cuffsockellinie 76 ist das Cuffelement 68 an das Topsheet 64 fixiert. An ihren jeweiligen Längsendbereichen sind die Cuffelemente 68 über schematisch angedeutete Fixierungen 77 auf das Topsheet 64 beziehungsweise auf sich selbst in einer C-förmig gefalteten Konfiguration festgelegt, wie in Figur 5b gezeigt.

Der erste Inkontinenzartikel 2 nach Figuren 1 bis 3 und Figur 4 links bildet mit dem zweiten Inkontinenzartikel 2' nach Figur 4 rechts eine beanspruchte Anordnung ("Array").

Ein solcher Array als eine gemeinsame Darstellung eines ersten Inkontinenzartikel 2 mit Cuffelementen ohne Elastifizierung und eines zweiten Inkontinenzartikel 2' mit Cuffelementen mit Elastifizierung von ein und demselben Hersteller ist für unterschiedliche Benutzungssituationen vorgesehen, wie eben leichtere Inkontinenz und stärkere Inkontinenz. Hierfür können sich die beiden Inkontinenzartikel vorzugsweise in den Dimensionen des Schrittabschnitts und des Absorptionskörpers unterscheiden. Weiter können auch die Cuffhöhen H und vorzugsweise weiter auch Dimensionsverhältnisse von Cuffhöhe H zur Querstreckung F des Schrittabschnitts als auch von Cuffhöhe H zum Abstand G zwischen Cuffsockellinie und den Schrittelastifizierungsmittel, mit H, F und G gemessen an schmalster Stelle des Schrittabschnitts, sich unterscheiden. In Tabelle 1 ist ein Beispiel eines solches Arrays für die Größe M dargestellt.

| | | Erster Inkontinenzartikel | Zweiter Inkontinenzartikel |
|---|---|---|---|
| | | Größe M | Größe M |
| Cuffelement | | Ohne jegliche Elastifizierung | Mit Elastifizierungsmittel im freien Längsrand |
| Fläche Schrittabschnitt | mm² | 63.000-66.000 | 133.000-137.000 |
| Fläche Absorptionskörper | mm² | 32.000-36.000 | 79.000-82.000 |
| Längserstreckung Schrittabschnitt | mm | 370-410 | 550-580 |
| Maximale Quererstreckung Schrittabschnitt | mm | 150-180 | 230-270 |
| Längserstreckung Absorptionskörper | mm | 320-360 | 470-510 |
| Maximale Quererstreckung Absorptionskörper | mm | 100-140 | 160-200 |
| | | | |
| Cuffhöhe H | mm | 10-25 | 25-35 |
| H/F | | 0,4-0,6 | 0,8-1,1 |
| H/G | | 0,7-1,0 | 1,1-1,4 |

## Patentansprüche

1. Inkontinenzartikel (2) in Höschenform für die Aufnahme von Körperausscheidungen mit einem vorderen Bauchabschnitt (4) und einem hinteren Rückenabschnitt (6), die in einer Längsrichtung (9) voneinander beabstandet sind und die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, und
mit einem einen Absorptionskörper (7) aufweisenden Schrittabschnitt (8), der sich in der Längsrichtung (9) zwischen Bauchabschnitt (4) und Rückenabschnitt (6) erstreckt und an den Bauchabschnitt (4) und an den Rückenabschnitt (6) in einem jeweiligen Überlappungsbereich (36, 38) unlösbar angefügt ist,
- wobei der Schrittabschnitt (8), der Bauchabschnitt (4) und der Rückenabschnitt (6) gemeinsam jeweilige Beinöffnungen (19) des Inkontinenzartikels begrenzen,
- wobei der Bauchabschnitt (4) und der Rückenabschnitt (8) in Quer- oder Hüftumfangsrichtung (16) elastisch dehnbar ausgebildet sind,
- wobei der Schrittabschnitt (8) beidseits eine seitliche Auslaufsperre bildende und beidseits entlang der Längserstreckung des Absorptionskörpers (7) verlaufende Cuffelemente (68) aufweist, die zumindest entlang einer Cuffsockellinie (76) an der körperzugewandten Seite des Artikels festgelegt sind und einen unbefestigten freien Längsrand (74) aufweisen, mit dem sie sich von der körperzugewandten Seite des Artikels emporerheben können und so die jeweilige seitliche Auslaufsperre bilden,
- wobei die Cuffelemente (68) aus einem auf sich selbst entlang der Längsrichtung (9) umgefalteten Vliesmaterialabschnitt (70) gebildet sind,
- wobei eine hierbei entstehende Faltlinie (72) den unbefestigten freien Längsrand (74) bildet,
- wobei die Cuffsockellinie (76) ganz in dem umgefalteten Bereich (78) des Vliesmaterialabschnitts (70) verläuft,
- wobei der Schrittabschnitt (8) außerhalb des Absorptionskörpers (7) und beidseits des Absorptionskörpers im Wesentlichen in der Längsrichtung (9) verlaufende und der jeweiligen Beinöffnung (19) zugeordnete Schrittelastifizierungsmittel (84) aufweist,
- wobei die Cuffelemente (68) in Querrichtung (16) innerhalb der jeweiligen Cuffsockellinie (76), also in einem gesamten von der körperzugewandten Seite des Artikels emporerhebbaren Bereich (80) frei von elastifizierenden Mitteln sind, und
- wobei die der jeweiligen Beinöffnung (19) zugeordneten Schrittelastifizierungsmittel (84) in Querrichtung (16) außerhalb der Cuffsockellinie (76) angeordnet sind.

2. Inkontinenzartikel (2) nach Anspruch 1, **dadurch gekennzeichnet, dass** eine Quererstreckung (H) des von der körperzugewandten Seite des Artikels emporerhebbaren Bereichs des jeweiligen Cuffelements (68) wenigstens das 0,3-fache, insbesondere wenigstens das 0,4-fache und höchstens das 1,0-fache, insbesondere höchstens das 0,9-fache, insbesondere höchstens das 0,8-fache und weiter insbesondere höchstens das 0,7-fache einer Quererstreckung (F) des Schrittabschnitts (8) gemessen von der Cuffsockellinie (76) nach außen beträgt, wobei die jeweilige Quererstreckung (H, F) an einer in Querrichtung (16) schmalsten Stelle des Schrittabschnitts (8) bestimmt wird, die in der Längsrichtung (9) zwischen dem Bauchabschnitt (4) und dem Rückenabschnitt (6) liegt.

3. Inkontinenzartikel (2) nach Anspruch 1 oder 2, **dadurch gekennzeichnet, dass** eine maximale Quererstreckung (H) des von der körperzugewandten Seite des Artikels emporerhebbaren Bereichs (80) des jeweiligen Cuffelements (68) wenigstens 7 mm, insbesondere wenigstens 10 mm, insbesondere wenigstens 12 mm und höchstens 30 mm, insbesondere höchstens 25 mm, insbesondere höchstens 20 mm und weiter insbesondere höchstens 18 mm und insbesondere 15 mm beträgt, wobei die jeweilige Quererstreckung (H) an einer Stelle des Schrittabschnitts (8) bestimmt wird, die in der Längsrichtung (9) zwischen dem Bauchabschnitt (4) und dem Rückenabschnitt (6) liegt.

4. Inkontinenzartikel (2) nach Anspruch 1, 2 oder 3, **dadurch gekennzeichnet, dass** eine Quererstreckung (H) des von der körperzugewandten Seite des Artikels emporerhebbaren Bereichs (80) des jeweiligen Cuffelements (68) wenigstens das 0,50-fache, insbesondere wenigstens das 0,55-fache, insbesondere wenigstens das 0,60-fache, insbesondere wenigstens das 0,65-fache und höchstens das 1,0-fache, insbesondere höchstens das 0,95-fache, insbesondere höchstens das 0,90-fache eines Querabstands (G) zwischen der Cuffsockellinie (76) und eines in Querrichtung (16) äußersten Schrittelastifizierungsmittels (84) beträgt, wobei die Quererstreckung (H) und der Querabstand (G) an einer in Querrichtung (16) schmalsten Stelle des Schrittabschnitts (8) bestimmt wird, die in der Längsrichtung (9) zwischen dem Bauchabschnitt (4) und dem Rückenabschnitt (6) liegt.

5. Inkontinenzartikel (2) nach einem oder mehreren der vorstehenden Ansprüche, **dadurch gekennzeichnet, dass** eine Quererstreckung (H) des von der körperzugewandten Seite des Artikels emporerhebbaren Bereichs (80) des jeweiligen Cuffelements (68) höchstens das 5,0-fache, insbesondere höchstens das 4,5-fache, insbesondere höchstens das 4,0-fache und wenigstens das 1,0-fache, insbesondere wenigstens das 1,5-fache, weiter insbesondere wenigstens das 2,0-fache einer Dicke des Absorptionskörpers (7) beträgt, wobei die Quererstreckung (H) an einer in Querrichtung (16) schmalsten Stelle des Schrittabschnitts (8) bestimmt wird, die in der Längsrichtung (9) zwischen dem Bauchabschnitt (4) und dem Rückenabschnitt (6) liegt.

6. Inkontinenzartikel (2) nach einem oder mehreren der vorstehenden Ansprüche, **dadurch gekennzeichnet, dass** die aufeinandergefalteten Bereiche (78) des Vliesmaterialabschnitts (70) in dem gesamten von der körperzugewandten Seite des Artikels emporerhebbaren Bereich (78) nur lose gegeneinander anliegen, also frei von Fügestellen sind.

7. Inkontinenzartikel (2) nach einem oder mehreren der vorstehenden Ansprüche, **dadurch gekennzeichnet, dass** der emporerhebbare Bereich (80) des jeweiligen Cuffelements (68) nicht über Fügestellen mit in Querrichtung (16) außerhalb der Cuffsockellinie (76) liegenden Chassismaterialien oder Komponenten des Schrittabschnitts (8) verbunden ist.

8. Inkontinenzartikel (2) nach einem oder mehreren der vorstehenden Ansprüche, **dadurch gekennzeichnet, dass** die jeweilige Cuffsockellinie (76) hinten und/oder vorn einen in Querrichtung (16), insbesondere bogenförmig nach innen, also in Richtung auf den unbefestigten freien Längsrand (74) verlaufenden Cuffsockellinienabschnitt (82) aufweist, welcher den emporerhebbaren Bereich (80) des Cuffelements (68) in der Längsrichtung (9) begrenzt.

9. Inkontinenzartikel (2) nach einem oder mehreren der vorstehenden Ansprüche, **dadurch gekennzeichnet, dass** die jeweilige Cuffsockellinie (76) über ihre im Wesentlichen gesamte Längserstreckung gerade erstreckt ist, und hiervon ausgehend hinten und/oder vorn einen zusätzlichen in Querrichtung (16), insbesondere bogenförmig nach innen, also in Richtung auf den unbefestigten freien Längsrand verlaufenden Cuffsockellinienabschnitt (82) aufweist oder aufweisen kann, welcher den emporerhebbaren Bereich (80) des Cuffelements (68) in der Längsrichtung (9) begrenzt.

10. Inkontinenzartikel (2) nach einem oder mehreren der vorstehenden Ansprüche, **dadurch gekennzeichnet, dass** die jeweilige Cuffsockellinie (76) innerhalb eines in der Längsrichtung (9) mittleren Bereichs des Schrittabschnitts (8) außerhalb des Absorptionskörpers (7) verläuft und in einem vorderen und/oder hinteren Bereich des Schrittabschnitts (8) auf dem Absorptionskörper (7) verläuft.

11. Inkontinenzartikel (2) nach einem oder mehreren der vorstehenden Ansprüche, **dadurch gekennzeichnet, dass** eine Längserstreckung des Schrittabschnitts (8) wenigstens das 0,45-fache, insbesondere wenigstens das 0,50-fache und höchstens das 0,70-fache, insbesondere höchstens das 0,65-fache, insbesondere höchstens das 0,60-fache einer Gesamtlängserstreckung des Inkontinenzartikels beträgt.

12. Inkontinenzartikel (2) nach einem oder mehreren der vorstehenden Ansprüche, **dadurch gekennzeichnet, dass** ein vorderes und/oder hinteres Ende des Schrittabschnitts (8) und jedenfalls ein jeweiliges vorderes und/oder hinteres Längsende des Absorptionskörpers (7) des Schrittabschnitts (8) in der Längsrichtung (9) vor einem schrittseitigen Ende der Seitennahtbereiche (14) endet.

13. Inkontinenzartikel (2) nach einem oder mehreren der vorstehenden Ansprüche, **dadurch gekennzeichnet, dass** eine Fläche des Schrittabschnitts (8) im eben ausgebreiteten Zustand 12 - 30 %, insbesondere 15 - 25 %, insbesondere 15 - 23 % einer Produktgesamtfläche des Inkontinenzartikels beträgt.

14. Inkontinenzartikel (2) nach einem oder mehreren der vorstehenden Ansprüche, **dadurch gekennzeichnet, dass** eine Fläche des Absorptionskörpers (7) im eben ausgebreiteten Zustand 7 - 15 %, insbesondere 8 - 13 % einer Produktgesamtfläche des Inkontinenzartikels beträgt.

15. Inkontinenzartikel (2) nach einem oder mehreren der vorstehenden Ansprüche, **dadurch gekennzeichnet, dass** eine Fläche des Schrittabschnitts (8) im eben ausgebreiteten Zustand 60.000 - 80.000 mm², insbesondere 60.000 - 75.000 mm², weiter insbesondere 62.000 - 72.000 mm² und/oder eine Fläche des Absorptionskörpers (7) im eben ausgebreiteten Zustand 30.000 - 45.000 mm², insbesondere 30.000 - 42.000 mm², weiter insbesondere 32.000 - 40.000 mm² beträgt.

16. Inkontinenzartikel (2) nach einem oder mehreren der vorstehenden Ansprüche, **dadurch gekennzeichnet, dass** der Schrittabschnitt (8) 4 - 15 %, insbesondere 4 - 12 %, insbesondere 4 - 10 % der Fläche des Bauchabschnitts (4) überlappt und/oder dass der Schrittabschnitt (8) 5 - 20 %, insbesondere 5 - 16 %, insbesondere 5 - 14 %, insbesondere 5 - 12 % der Fläche des Rückenabschnitts (6) überlappt.

17. Inkontinenzartikel (2) nach einem oder mehreren der vorstehenden Ansprüche, **dadurch gekennzeichnet, dass** eine maximale Quererstreckung des Schrittabschnitts (8) wenigstens das 0,15-fache, insbesondere wenigstens das 0,18-fache, insbesondere wenigstens das 0,20-fache und höchstens das 0,30-fache, insbesondere höchstens das 0,28-fache einer maximalen Quererstreckung des Inkontinenzartikels beträgt.

18. Inkontinenzartikel (2) nach einem oder mehreren der vorstehenden Ansprüche, **dadurch gekennzeichnet, dass** der Schrittabschnitt (8) ein flüssigkeitsundurchlässiges Backsheet-Material (62) und ein flüssigkeitsdurchlässiges Topsheet-Material (64) umfasst, zwischen denen der Absorptionskörper (7) angeordnet ist.

19. Inkontinenzartikel (2) nach einem oder mehreren der vorstehenden Ansprüche, **dadurch gekennzeichnet, dass** eine Längserstreckung der jeweiligen Seitennahtbereiche (14) das 0,18 - 0,30-fache, insbesondere 0,0,18 - 0,28-fache, insbesondere das 0,18 - 0,25-fache einer maximalen Quererstreckung des Inkontinenzartikels beträgt.

20. Inkontinenzartikel (2) nach einem oder mehreren der vorstehenden Ansprüche, **dadurch gekennzeichnet, dass** in dem Bauchabschnitt (4) und/oder in dem Rückenabschnitt (6) erste Elastifizierungsmittel (28) vorgesehen sind, die sich in einem Abstand voneinander und im Wesentlichen in Quer- oder Hüftumfangsrichtung (16) erstrecken und so den Bauchabschnitt (4) und/oder den Rückenabschnitt (6) flächenhaft elastifizieren, oder dass der Bauchabschnitt (4) und/oder der Rückenabschnitt (6) in Quer- oder Hüftumfangsrichtung elastisch dehnbare Flächenmaterialien umfasst.

21. Anordnung (Array) aus einem ersten Inkontinenzartikel (2) nach einem oder mehreren der vorstehenden Ansprüche und einem zweiten Inkontinenzartikel (2') in Höschenform für die Aufnahme von Körperausscheidungen mit einem vorderen Bauchabschnitt (4) und einem hinteren Rückenabschnitt (6), die in einer Längsrichtung (9) voneinander beabstandet sind und die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), der sich in der Längsrichtung (9) zwischen Bauchabschnitt (4) und Rückenabschnitt (6) erstreckt und an den Bauchabschnitt (4) und an den Rückenabschnitt (6) in einem jeweiligen Überlappungsbereich (34, 36) unlösbar angefügt ist, wobei der Schrittabschnitt (8), der Bauchabschnitt (4) und der Rückenabschnitt (6) gemeinsam jeweilige Beinöffnungen (19) des Inkontinenzartikels begrenzen, wobei der Bauchabschnitt (4) und der Rückenabschnitt (6) in Quer- oder Hüftumfangsrichtung (16) elastisch dehnbar ausgebildet sind, wobei der Schrittabschnitt (8) beidseits eine seitliche Auslaufsperre bildende und beidseits entlang der Längserstreckung des Absorptionskörpers (7) verlaufende Cuffelemente (68) aufweist, die zumindest entlang einer Cuffsockellinie (76) an der körperzugewandten Seite des Artikels festgelegt sind und einen unbefestigten freien Längsrand (74) aufweisen, mit dem sie sich von der körperzugewandten Seite des Artikels emporerheben können und so die jeweilige seitliche Auslaufsperre bilden,
wobei der Schrittabschnitt (8) außerhalb des Absorptionskörpers (7) und beidseits des Absorptionskörpers (7) in der Längsrichtung (9) verlaufende und der jeweiligen Beinöffnung zugeordnete Schrittelastifizierungsmittel (82) aufweist,
wobei das jeweilige Cuffelement (76) des zweiten Inkontinenzartikels (2') in einem von der körperzugewandten Seite des Artikels emporerhebbaren Bereich, insbesondere entlang seines unbefestigten freien Längsrands, in der Längsrichtung elastisch oder durch Elastifizierungsmittel (100) elastifiziert ausgebildet ist.

22. Anordnung nach Anspruch 21, **dadurch gekennzeichnet, dass** eine Längserstreckung und eine Quererstreckung des Schrittabschnitts (8) und des Absorptionskörpers (7) des zweiten Inkontinenzartikels (2') größer ist als eine Längserstreckung und eine Quererstreckung des Schrittabschnitts (8) und des Absorptionskörpers (7) des ersten Inkontinenzartikels (2).

23. Anordnung (Array) nach Anspruch 21 oder 22, **dadurch gekennzeichnet, dass** der erste und der zweite Inkontinenzartikel (2, 2') für die Verwendung durch Frauen vorgesehen ist.

## Claims

1. An incontinence product (2) in the form of briefs for the absorption of bodily excretions, having a front abdominal portion (4) and a rear back portion (6) which are spaced apart from one another in a longitudinal direction (9) and which are connected to each other at side seam regions (14) on both sides during production, to form an abdominal and back bandcontinuous in the transverse or hip circumference direction (16) with a hip opening (18) which is closed in the hip circumference direction, and
having a crotch portion (8) which comprises an absorption body (7) and extends in the longitudinal direction (9) between the abdominal portion (4) and the back portion (6) and is unreleasably joined to the abdominal portion (4) and to the back portion (6) in a respective overlap region (36, 38),
- wherein the crotch portion (8), the abdominal portion (4) and the back portion (6) together delimit respective leg openings (19) of the incontinence product,
- wherein the abdominal portion (4) and the back portion (8) are realized so as to be elastically expandable in the transverse or hip circumference direction (16),
- wherein the crotch portion (8) comprises on both sides cuff elements (68), which form a lateral anti-leak barrier, extend on both sides along the longitudinal extent of the absorption body (7), are fixed to the side of the product facing the body at least along a cuff base line (76) and comprise a non-fastened free longitudinal edge (74) by way of which they are able to be raised from the side of the product facing the body and thus form the respective lateral anti-leak barrier,
- wherein the cuff elements (68) are formed from a non-woven material portion (70) which is folded around onto itself along the longitudinal direction (9),
- wherein a folding line (72) thus generated forms the non-fastened free longitudinal edge (74),
- wherein the cuff base line (76) extends entirely in the folded-around region (78) of the non-woven material portion (70),
- wherein the crotch portion (8) comprises crotch elasticizing means (84) which extend outside the absorption body (7) and on both sides of the absorption body substantially in the longitudinal direction (9) and are assigned to the respective leg opening (19),
- wherein the cuff elements (68) are free of elasticizing means in the transverse direction (16) inside the respective cuff base line (76), that is to say in an entire region (80) which is able to be raised from the side of the product facing the body, and
- wherein the crotch elasticizing means (84) assigned to the respective leg opening (19) are arranged outside the cuff base line (76) in the transverse direction (16) .

2. The incontinence product (2) as claimed in claim 1, **characterized in that** a transverse extent (H) of the region of the respective cuff element (68) which is able to be raised from the side of the product facing the body is at least 0.3 times, in particular at least 0.4 times and no more than 1.0 times, in particular no more than 0.9 times, in particular no more than 0.8 times and further in particular no more than 0.7 times a transverse extent (F) of the crotch portion (8), measured outward from the cuff base line (76), wherein the respective transverse extent (H, F) is determined at a position of the crotch portion (8) which is narrowest in the transverse direction (16) and lies between the abdominal portion (4) and the back portion (6) in the longitudinal direction (9).

3. The incontinence product (2) as claimed in claim 1 or 2, **characterized in that** a maximum transverse extent (H) of the region (80) of the respective cuff element (68) which is able to be raised from the side of the product facing the body is at least 7 mm, in particular at least 10 mm, in particular at least 12 mm and no more than 30 mm, in particular no more than 25 mm, in particular no more than 20 mm and further in particular no more than 18 mm and in particular 15 mm, wherein the respective transverse extent (H) is determined at a position of the crotch portion (8) which lies between the abdominal portion (4) and the back portion (6) in the longitudinal direction (9).

4. The incontinence product (2) as claimed in claim 1, 2 or 3, **characterized in that** a transverse extent (H) of the region (80) of the respective cuff element (68) which is able to be raised from the side of the product facing the body is at least 0.50 times, in particular at least 0.55 times, in particular at least 0.60 times, in particular at least 0.65 times and no more than 1.0 times, in particular no more than 0.95 times, in particular no more than 0.90 times a transverse distance (G) between the cuff base line (76) and a crotch elasticizing means (84) that is outermost in the transverse direction (16), wherein the transverse extent (H) and the transverse distance (G) are determined at a position of the crotch portion (8) which is narrowest in the transverse direction (16) and lies between the abdominal portion (4) and the back portion (6) in the longitudinal direction (9).

5. The incontinence product (2) as claimed in one or several of the preceding claims, **characterized in that** a transverse extent (H) of the region (80) of the respective cuff element (68) which is able to be raised from the side of the product facing the body is no more than 5.0 times, in particular no more than 4.5 times, in particular no more than 4.0 times and at least 1.0 times, in particular at least 1.5 times, further in particular at least 2.0 times a thickness of the absorption body (7), wherein the transverse extent (H) is determined at a position of the crotch portion (8) which is narrowest in the transverse direction (16) and lies between the abdominal portion (4) and the back portion (6) in the longitudinal direction (9).

6. The incontinence product (2) as claimed in one or several of the preceding claims, **characterized in that** the regions (78) of the non-woven material portion (70) folded onto one another only abut loosely against one another in the entire region (78) which is able to be raised from the side of the product facing the body, that is to say are free of joints.

7. The incontinence product (2) as claimed in one or several of the preceding claims, **characterized in that** the region (80) of the respective cuff element (68) which is able to be raised is not connected via joints to chassis materials or components of the crotch portion (8) lying outside the cuff base line (76) in the transverse direction (16).

8. The incontinence product (2) as claimed in one or several of the preceding claims, **characterized in that** the respective cuff base line (76) comprises to the rear and/or to the front a cuff base line portion (82) which extends in the transverse direction (16) inwards, in particular inwards in an arcuate manner, that is to say in the direction of the non-fastened free longitudinal edge (74), and delimits the raisable region (80) of the cuff element (68) in the longitudinal direction (9).

9. The incontinence product (2) as claimed in one or several of the preceding claims, **characterized in that** the respective cuff base line (76) extends in a straight manner over its substantially entire longitudinal extent, and proceeding from here to the rear and/or to the front, comprises or can comprise an additional cuff base line portion (82) which extends in the transverse direction (16) inwards, in particular inwards in an arcuate manner, that is to say in the direction of the non-fastened free longitudinal edge, and delimits the raisable region (80) of the cuff element (68) in the longitudinal direction (9).

10. The incontinence product (2) as claimed in one or several of the preceding claims, **characterized in that** the respective cuff base line (76) extends inside a region of the crotch portion (8) that is central in the longitudinal direction (9) outside the absorption body (7) and extends in a front and/or rear region of the crotch portion (8) on the absorption body (7).

11. The incontinence product (2) as claimed in one or several of the preceding claims, **characterized in that** a longitudinal extent of the crotch portion (8) is at least 0.45 times, in particular at least 0.50 times and no more than 0.70 times, in particular no more than 0.65 times, in particular no more than 0.60 times an overall longitudinal extent of the incontinence product.

12. The incontinence product (2) as claimed in one or several of the preceding claims, **characterized in that** a front and/or rear end of the crotch portion (8) and at any rate a respective front and/or rear longitudinal end of the absorption body (7) of the crotch portion (8) ends in front of a crotch-side end of the side seam regions (14) in the longitudinal direction (9).

13. The incontinence product (2) as claimed in one or several of the preceding claims, **characterized in that** a surface of the crotch portion (8) in the state spread out flatly is 12 - 30 %, in particular 15 - 25 %, in particular 15 - 23 % of an entire product surface of the incontinence product.

14. The incontinence product (2) as claimed in one or several of the preceding claims, **characterized in that** a surface of the absorption body (7) in the state spread out flatly is 7 - 15 %, in particular 8 - 13 % of an entire product surface of the incontinence product.

15. The incontinence product (2) as claimed in one or several of the preceding claims, **characterized in that** a surface of the crotch portion (8) in the state spread out flatly is 60,000 - 80,000 mm², in particular 60,000 - 75,000 mm², further particularly 62,000 - 72,000 mm² and/or a surface of the absorption body (7) in the state spread out flatly is 30,000 - 45,000 mm², in particular 30,000 - 42,000 mm² and further particularly 32,000 - 40,000 mm².

16. The incontinence product (2) as claimed in one or several of the preceding claims, **characterized in that** the crotch portion (8) overlaps 4 - 15 %, in particular 4 - 12 %, in particular 4 - 10 % of the surface of the abdominal portion (4) and/or **in that** the crotch portion (8) overlaps 5 - 20 %, in particular 5 - 16 %, in particular 5 - 14 %, in particular 5 - 12 % of the surface of the back portion (6).

17. The incontinence product (2) as claimed in one or several of the preceding claims, **characterized in that** a maximum transverse extent of the crotch portion (8) is at least 0.15 times, in particular at least 0.18 times, in particular at least 0.20 times and no more than 0.30 times, in particular no more than 0.28 times a maximum transverse extent of the incontinence product.

18. The incontinence product (2) as claimed in one or several of the preceding claims, **characterized in that** the crotch portion (8) includes a fluid-impermeable back sheet material (62) and a fluid-permeable top sheet material (64), between which the absorption body (7) is arranged.

19. The incontinence product (2) as claimed in one or several of the preceding claims, **characterized in that** a longitudinal extent of the respective side seam regions (14) is 0.18 - 0.30 times, in particular 0.18 - 0.28 times, in particular 0.18 - 0.25 times a maximum transverse extent of the incontinence product.

20. The incontinence product (2) as claimed in one or several of the preceding claims, **characterized in that** first elasticizing means (28) are provided in the abdominal portion (4) and/or in the back portion (6), which first elasticizing means (28) extend at a distance from one another and substantially in the transverse or hip circumference direction (16) and thus make the abdominal portion (4) and/or the back portion (6) extensively elasticized, or **in that** the abdominal portion (4) and/or the back portion (6) include surface materials which are elastically expandable in the transverse direction or in the direction of the hip circumference.

21. An arrangement (array) produced from a first incontinence product (2) as claimed in one or several of the preceding claims and a second incontinence product (2') in the form of briefs for the absorption of bodily excretions, having a front abdominal portion (4) and a rear back portion (6) which are spaced apart from one another in a longitudinal direction (9) and which are connected to each other at side seam regions (14) on both sides during production, to form an abdominal and back band continuous in the transverse or hip circumference direction (16) with a hip opening (18) which is closed in the hip circumference direction, and having a crotch portion (8) which comprises an absorption body (7) and extends in the longitudinal direction (9) between the abdominal portion (4) and the back portion (6) and is unreleasably joined to the abdominal portion (4) and to the back portion (6) in a respective overlap region (34, 36),
wherein the crotch portion (8), the abdominal portion (4) and the back portion (6) together delimit respective leg openings (19) of the incontinence product,
wherein the abdominal portion (4) and the back portion (6) are realized so as to be elastically expandable in the transverse or hip circumference direction (16), wherein the crotch portion (8) comprises on both sides cuff elements (68), which form a lateral anti-leak barrier, extend on both sides along the longitudinal extent of the absorption body (7), are fixed to the side of the product facing the body at least along a cuff base line (76) and comprise a non-fastened free longitudinal edge (74) by way of which they are able to be raised from the side of the product facing the body and thus form the respective lateral anti-leak barrier, wherein the crotch portion (8) comprises crotch elasticizing means (82) which extend outside the absorption body (7) and on both sides of the absorption body (7) in the longitudinal direction (9) and are assigned to the respective leg opening,
wherein the respective cuff element (76) of the second incontinence product (2'), in a region which is able to be raised from the side of the product facing the body, in particular along its non-fastened free longitudinal edge, is realized so as to be elastic in the longitudinal direction or so as to be elasticized by elasticizing means (100).

22. The arrangement as claimed in claim 21, **characterized in that** a longitudinal extent and a transverse extent of the crotch portion (8) and of the absorption body (7) of the second incontinence product (2') are greater than a longitudinal extent and a transverse extent of the crotch portion (8) and of the absorption body (7) of the first incontinence product (2).

23. The arrangement (array) as claimed in claim 21 or 22, **characterized in that** the first and the second incontinence products (2, 2') are provided for use by women.

## Revendications

1. Article d'incontinence (2) sous forme de culotte, destiné à recueillir les excrétions corporelles, comprenant une portion ventrale avant (4) et une portion dorsale arrière (6) qui sont espacées l'une de l'autre dans une direction longitudinale (9) et qui, pour la formation d'une bande ventrale et dorsale continue dans le sens transversal ou dans le sens du tour de hanches (16), avec une ouverture de hanche (18) fermée dans le sens du tour de hanches, sont reliées entre elles par le fabricant au niveau de zones de couture latérale (14) situées de part et d'autre, et
comprenant une portion d'entrejambe (8) qui présente un corps absorbant (7) et s'étend dans la direction longitudinale (9) entre la portion ventrale (4) et la portion dorsale (6) et qui est rapportée de manière inamovible sur la portion ventrale (4) et sur la portion dorsale (6) dans une zone de chevauchement (36, 38) respective
- dans lequel ladite portion d'entrejambe (8), ladite portion ventrale (4) et ladite portion dorsale (6) délimitent ensemble des ouvertures de jambe (19) respectives de l'article d'incontinence,
- dans lequel la portion ventrale (4) et la portion dorsale (6) sont réalisées de manière à être élastiquement extensibles dans le sens transversal ou dans le sens du tour de hanches,
- dans lequel la portion d'entrejambe (8) comprend des éléments de manchette (68) qui forment une barrière anti-fuites latérale des deux côtés et s'étendent des deux côtés le long de l'extension longitudinale du corps absorbant (7) et qui sont fixés au moins le long d'une ligne de base de manchette (76) sur le côté de l'article, qui est tourné vers le corps, et présentent un bord longitudinal libre (74) non fixé avec lequel ils peuvent s'étendre vers le haut depuis le côté de l'article, qui est tourné vers le corps, et forment ainsi la barrière anti-fuites latérale respective,
- dans lequel les éléments de manchette (68) sont formés à partir d'un tronçon de matériau non tissé (70) replié sur lui-même le long de la direction longitudinale (9),
- dans lequel une ligne de pliage (72) ainsi créée forme le bord longitudinal libre (74) non fixé,
- dans lequel la ligne de base de manchette (76) s'étend entièrement dans la zone repliée (78) du tronçon de matériau non tissé (70),
- dans lequel la portion d'entrejambe (8) comprend des moyens d'élastification d'entrejambe (84) à l'extérieur du corps absorbant (7) et de part et d'autre du corps absorbant, qui s'étendent pour l'essentiel dans la direction longitudinale (9) et sont associés à l'ouverture de jambe (19) respective,
- dans lequel les éléments de manchette (68) sont dépourvus de moyens d'élastification dans le sens transversal (16) à l'intérieur de la ligne de base de manchette (76) respective, donc dans une zone entière (80) qui peut s'étendre vers le haut depuis le côté de l'article, qui est tourné vers le corps, et
- dans lequel les moyens d'élastification d'entrejambe (84) associés à l'ouverture de jambe (19) respective sont disposés à l'extérieur de la ligne de base de manchette (76) dans le sens transversal (16).

2. Article d'incontinence (2) selon la revendication 1, **caractérisé par le fait qu'**une extension transversale (H) de la zone de l'élément de manchette (68) respectif, qui peut s'étendre vers le haut depuis le côté de l'article, qui est tourné vers le corps, fait au moins 0,3 fois, en particulier au moins 0,4 fois et 1,0 fois tout au plus, en particulier 0,9 fois tout au plus, en particulier 0,8 fois tout au plus et en outre en particulier 0,7 fois tout au plus d'une extension transversale (F) de la portion d'entrejambe (8), mesurée depuis la ligne de base de manchette (76) vers l'extérieur, dans lequel l'extension transversale respective (H, F) est déterminée sur un point de la portion d'entrejambe (8) qui est le plus étroit dans le sens transversal (16) et qui est situé dans la direction longitudinale (9) entre la portion ventrale (4) et la portion dorsale (6).

3. Article d'incontinence (2) selon la revendication 1 ou 2, **caractérisé par le fait qu'**une extension transversale maximale (H) de la zone (80) de l'élément de manchette respectif (68) qui peut s'étendre vers le haut depuis le côté de l'article, qui est tourné vers le corps mesure au moins 7 mm, en particulier au moins 10 mm, en particulier au moins 12 mm et 30 mm tout au plus, en particulier 25 mm tout au plus, en particulier 20 mm tout au plus et en outre en particulier 18 mm tout au plus et en particulier 15 mm, dans lequel l'extension transversale respective (H) est déterminée sur un point de la portion d'entrejambe (8) qui est situé dans la direction longitudinale (9) entre la portion ventrale (4) et la portion dorsale (6).

4. Article d'incontinence (2) selon la revendication 1, 2 ou 3, **caractérisé par le fait qu'**une extension transversale (H) de la zone (80) de l'élément de manchette respectif (68), qui peut s'étendre vers le haut depuis le côté de l'article, qui est tourné vers le corps, fait au moins 0,50 fois, en particulier au moins 0,55 fois, en particulier au moins 0,60 fois, en particulier au moins 0,65 fois et 1,0 fois tout au plus, en particulier 0,95 fois tout au plus, en particulier 0,9 fois tout au plus d'une distance transversale (G) entre la ligne de base de manchette (76) et un moyen d'élastification d'entrejambe (84) situé le plus à l'extérieur dans le sens transversal (16), dans lequel l'extension transversale (H) et la distance transversale (G) sont déterminées sur un point de la portion d'entrejambe (8) qui est le plus étroit dans le sens transversal (16) et qui se trouve dans la direction longitudinale (9) entre la portion ventrale (4) et la portion dorsale (6).

5. Article d'incontinence (2) selon une ou plusieurs des revendications précédentes, **caractérisé par le fait qu'**une extension transversale (H) de la zone (80) de l'élément de manchette (68) respectif, qui peut s'étendre vers le haut depuis le côté de l'article, qui est tourné vers le corps, fait 5,0 fois tout au plus, en particulier 4,5 fois tout au plus, en particulier 4,0 fois tout au plus et au moins 1,0 fois, en particulier au moins 1,5 fois, en outre en particulier au moins 2,0 fois d'une épaisseur du corps absorbant (7), dans lequel l'extension transversale (H) est déterminée sur un point de la portion d'entrejambe (8) qui est le plus étroit dans le sens transversal (16) et qui est situé dans la direction longitudinale (9) entre la portion ventrale (4) et la portion dorsale (6).

6. Article d'incontinence (2) selon une ou plusieurs des revendications précédentes, **caractérisé par le fait que**, dans toute la zone (78) apte à s'étendre vers le haut depuis le côté de l'article qui est tourné vers le corps, les zones (78) du tronçon de matériau non tissé (70) qui sont repliées les unes sur les autres s'appliquent les unes contre les autres sans être reliées, donc en étant exemptes de joints.

7. Article d'incontinence (2) selon une ou plusieurs des revendications précédentes, **caractérisé par le fait que** la zone (80) de l'élément de manchette (68) respectif, qui peut s'étendre vers le haut, n'est pas reliée au moyen de joints à des matériaux de châssis ou des composants de la portion d'entrejambe (8) qui sont situés à l'extérieur de la ligne de base de manchette (76) dans le sens transversal (16).

8. Article d'incontinence (2) selon une ou plusieurs des revendications précédentes, **caractérisé par le fait que** la ligne de base de manchette (76) respective présente à l'arrière et/ou à l'avant une portion de ligne de base de manchette (82) qui s'étend dans le sens transversal (16), en particulier en forme d'arc vers l'intérieur, c'est-à-dire en direction du bord longitudinal libre (74) non fixé, et qui délimite dans la direction longitudinale (9) la zone (80) de l'élément de manchette (68) qui peut s'étendre vers le haut.

9. Article d'incontinence (2) selon une ou plusieurs des revendications précédentes, **caractérisé par le fait que** la ligne de base de manchette (76) respective s'étend de manière droite sur toute son extension longitudinale pour l'essentiel, et présente ou peut présenter à partir de ceci, à l'arrière et/ou à l'avant, une portion de ligne de base de manchette (82) supplémentaire qui s'étend dans le sens transversal (16), en particulier en forme d'arc vers l'intérieur, c'est-à-dire en direction du bord longitudinal libre (74) non fixé, et qui délimite dans la direction longitudinale (9) la zone (80) de l'élément de manchette (68), qui peut s'étendre vers le haut.

10. Article d'incontinence (2) selon une ou plusieurs des revendications précédentes, **caractérisé par le fait que**, à l'intérieur d'une zone de la portion d'entrejambe (8), qui est centrale dans la direction longitudinale (9), la ligne de base de manchette (76) respective s'étend à l'extérieur du corps absorbant (7), et, dans une zone avant et/ou arrière de la portion d'entrejambe (8), elle s'étend sur le corps absorbant (7).

11. Article d'incontinence (2) selon une ou plusieurs des revendications précédentes, **caractérisé par le fait qu'**une extension longitudinale de la portion d'entrejambe (8) fait au moins 0,45 fois, en particulier au moins 0,50 fois et 0,70 fois tout au plus, en particulier 0,65 fois tout au plus, en particulier 0,60 fois tout au plus d'une extension longitudinale totale de l'article d'incontinence.

12. Article d'incontinence (2) selon une ou plusieurs des revendications précédentes, **caractérisé par le fait qu'**une extrémité avant et/ou arrière de la portion d'entrejambe (8) et en tout cas une extrémité longitudinale avant et/ou arrière respective du corps absorbant (7) de la portion d'entrejambe (8) se termine dans la direction longitudinale (9) devant une extrémité côté entrejambe des zones de couture latérale (14).

13. Article d'incontinence (2) selon une ou plusieurs des revendications précédentes, **caractérisé par le fait qu'**une surface de la portion d'entrejambe (8) à l'état déployé de manière plane est comprise entre 12 et 30 %, en particulier entre 15 et 25 %, en particulier entre 15 et 23 % d'une surface totale de produit de l'article d'incontinence.

14. Article d'incontinence (2) selon une ou plusieurs des revendications précédentes, **caractérisé par le fait qu'**une surface du corps absorbant (7) à l'état déployé de manière plane est comprise entre 7 et 15 %, en particulier entre 8 et 13 % d'une surface totale de produit de l'article d'incontinence.

15. Article d'incontinence (2) selon une ou plusieurs des revendications précédentes, **caractérisé par le fait qu'**une surface de la portion d'entrejambe (8) à l'état déployé de manière plane est comprise entre 60.000 et 80.000 mm², en particulier entre 60.000 et 75.000 mm², en outre en particulier entre 62.000 et 72.000 mm² et/ou qu'une surface du corps absorbant (7) à l'état déployé de manière plane est comprise entre 30.000 - 45.000 mm², en particulier entre 30.000 et 42.000 mm², en outre en particulier entre 32.000 et 40.000 mm².

16. Article d'incontinence (2) selon une ou plusieurs des revendications précédentes, **caractérisé par le fait que** la portion d'entrejambe (8) recouvre 4 à 15 %, en particulier 4 à 12 %, en particulier 4 à 10 % de la surface de la portion ventrale (4) et/ou que la portion d'entrejambe (8) recouvre 5 à 20 %, en particulier 5 à 16 %, en particulier 5 à 14 %, en particulier 5 à 12 % de la surface de la portion dorsale (6).

17. Article d'incontinence (2) selon une ou plusieurs des revendications précédentes, **caractérisé par le fait qu'**une extension transversale maximale de la portion d'entrejambe (8) fait au moins 0,15 fois, en particulier au moins 0,18 fois, en particulier au moins 0,20 fois et 0,30 fois tout au plus, en particulier 0,28 fois tout au plus d'une extension transversale maximale de l'article d'incontinence.

18. Article d'incontinence (2) selon une ou plusieurs des revendications précédentes, **caractérisé par le fait que** la portion d'entrejambe (8) comprend un matériau de feuille arrière imperméable aux liquides (62) et un matériau de feuille supérieure perméable aux liquides (64) entre lesquels est agencé ledit corps absorbant (7) .

19. Article d'incontinence (2) selon une ou plusieurs des revendications précédentes, **caractérisé par le fait qu'**une extension longitudinale des zones de couture latérale respectives (14) fait entre 0,18 et 0,30 fois, en particulier entre 0,18 et 0,28 fois, en particulier entre 0,18 et 0,25 fois d'une extension transversale maximale de l'article d'incontinence.

20. Article d'incontinence (2) selon une ou plusieurs des revendications précédentes, **caractérisé par le fait que** dans la portion ventrale (4) et/ou dans la portion dorsale (6) sont prévus des premiers moyens d'élastification (28) qui s'étendent à distance les uns des autres et pour l'essentiel dans le sens transversal ou dans le sens du tour de hanches (16) et qui élastifient ainsi en nappe la portion ventrale (4) et/ou la portion dorsale (6), ou que la portion ventrale (4) et/ou la portion dorsale (6) comprend des matériaux en nappe élastiquement extensibles dans le sens transversal ou dans le sens du tour de hanches.

21. Ensemble (array) se composant d'un premier article d'incontinence (2) selon une ou plusieurs des revendications précédentes et d'un deuxième article d'incontinence (2') sous forme de culotte, destiné à recueillir les excrétions corporelles, comprenant une portion ventrale avant (4) et une portion dorsale arrière (6) qui sont espacées l'une de l'autre dans une direction longitudinale (9) et qui, pour la formation d'une bande ventrale et dorsale continue dans le sens transversal ou dans le sens du tour de hanches (16), avec une ouverture de hanche (18) fermée dans le sens du tour de hanches, sont reliées entre elles par le fabricant au niveau de zones de couture latérale (14) situées de part et d'autre, ainsi qu'avec une portion d'entrejambe (8) qui présente un corps absorbant (7) et qui s'étend dans la direction longitudinale (9) entre la partie ventrale (4) et la partie dorsale (6) et qui est rapportée de manière inamovible sur la portion ventrale (4) et sur la portion dorsale (6) dans une zone de chevauchement (34, 36) respective,
dans lequel ladite portion d'entrejambe (8), ladite portion ventrale (4) et ladite portion dorsale (6) délimitent ensemble des ouvertures de jambe (19) respectives de l'article d'incontinence,
dans lequel la portion ventrale (4) et la portion dorsale (6) sont conçues pour être élastiquement extensibles dans le sens transversal ou dans le sens du tour de hanches (16), dans lequel la portion d'entrejambe (8) comprend des éléments de manchette (68) qui forment une barrière anti-fuites latérale des deux côtés et s'étendent des deux côtés le long de l'extension longitudinale du corps absorbant (7) et qui sont fixés au moins le long d'une ligne de base de manchette (76) sur le côté de l'article, qui est tourné vers le corps, et présentent un bord longitudinal libre (74) non fixé avec lequel ils peuvent s'étendre vers le haut depuis le côté de l'article, qui est tourné vers le corps, et forment ainsi la barrière anti-fuites latérale respective,
dans lequel la portion d'entrejambe (8) comprend des moyens d'élastification d'entrejambe (82) à l'extérieur du corps absorbant (7) et de part et d'autre du corps absorbant (7), qui s'étendent dans la direction longitudinale (9) et sont associés à l'ouverture de jambe (19) respective,
dans lequel l'élément de manchette respectif (76) du deuxième article d'incontinence (2') est conçu pour être élastique dans la direction longitudinale ou de manière élastifiée par des moyens d'élastification (100) dans une zone qui peut s'étendre vers le haut depuis le côté de l'article, qui est tourné vers le corps, en particulier le long de son bord longitudinal libre non fixé.

22. Ensemble selon la revendication 21, **caractérisé par le fait qu'**une extension longitudinale et une extension transversale de la portion d'entrejambe (8) et du corps absorbant (7) du deuxième article d'incontinence (2') est supérieure à une extension longitudinale et à une extension transversale de la portion d'entrejambe (8) et du corps absorbant (7) du premier article d'incontinence (2).

23. Ensemble (array) selon la revendication 21 ou 22, **caractérisé par le fait que** le premier et le deuxième article d'incontinence (2, 2') sont prévus pour être utilisés par des femmes.
